(19) Europäisches Patentamt
European Patent Office
Office européen des brevets

(11) EP 2 422 618 A1

(12) EUROPEAN PATENT APPLICATION

(43) Date of publication:
**29.02.2012 Bulletin 2012/09**

(51) Int Cl.:
***A01K 67/027*** *(2006.01)* ***A61K 49/00*** *(2006.01)*

(21) Application number: **10008952.3**

(22) Date of filing: **27.08.2010**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(71) Applicant: **Technologie Integrale Ltd.**
**Ramsey**
**Isle of Man IM8 1RD (GB)**

(72) Inventor: **The designation of the inventor has not yet been filed**

(74) Representative: **Lahrtz, Fritz**
**Isenbruck Bösl Hörschler LLP**
**Patentanwälte**
**Prinzregentenstrasse 68**
**81675 München (DE)**

# (54) Animal model for the evaluation of the efficacy of an HIV vaccine

(57) The present invention relates to the use of a Severe Combined T-B-Immune Deficient (SCID) mouse engrafted with human immunocompetent cells (huSCID-mouse) as an animal model for the evaluation of the effectiveness of an HIV vaccine. Furthermore, the present invention relates to a method for the evaluation of an HIV vaccine, wherein a huSCID-mouse of the invention is inoculated with the HIV vaccine and thereafter challenged with HI-virus.

EP 2 422 618 A1

## Description

The present application relates to an animal model for the evaluation of the efficacy of an HIV vaccine as well as to methods for the evaluation of the efficacy of an HIV vaccine using said animal model.

Animal models for studying HIV infection are known in the art. These models include monkeys, and also SCID mice optionally engrafted with human leukocytes. However, in the past, the animal models used for studying preclinical assays of HIV vaccine forms were run only in a very limited number of animal models, mostly in two models only - Macaque Rhesus and Chimpanzees. In macaque rhesus human HIV infection does not work, so vaccines were tested exclusively for eliciting HIV-specific antibodies response with extremely sensitive ELISPOT kit's assays. In chimps that are bearing HIV infection but without developing any symptoms of immune deficiency disease (AIDS) finally tests results were resumed to the same ELISPOT data.

Therefore, there is a need for an efficient animal model for testing HIV vaccines.

## Summary of the Invention

In one aspect, the present invention relates to the use of a Severe Combined T-B-Immune Deficient (SCID) mouse engrafted with human immunocompetent cells (huSCID-mouse) as an animal model for the evaluation of the effectiveness of an HIV vaccine.

In a preferred embodiment, the immunocompetent cells are able to develop a human-type immune reaction for HIV.

In a further preferred embodiment, the human immunocompetent cells are PBMC, dendritic cells or a mixture of PBMC and dendritic cells.

In a further preferred embodiment, the dendritic cells have been obtained by cultering human PBMC in the presence of cytokines capable of inducing the formation of dendritic cells in vitro. Preferably, the cytokines are also capable of cell differentiation blockage.

In a preferred embodiment, the human immunocompetent cells are derived from one human donor.

In a preferred embodiment, the mouse has been engrafted with 5-15x$10^6$ cells.

In a preferred embodiment, the huSCIDmouse is the particular breed of animals deficient in their own endogenous immune system and introduced with human immunocompetent cells or any other human cells.

In another aspect, the invention relates to a method for the evaluation of an HIV vaccine, wherein a huSCID-mouse of the invention is inoculated with the HIV vaccine and thereafter challenged with HI-virus.

In a preferred embodiment, the HIV vaccine is an HIV-1 envelop peptides/proteins cocktail, preferably wherein the proteins or peptides are of recombinant origin.

In a preferred embodiment, the evaluation of the vaccine is determined by determining the efficacy of the vaccine.

In a preferred embodiment, the efficacy is determined by determining the protection to HIV challenge.

In a preferred embodiment, the huSCID-mouse is inoculated with the HIV vaccine 1 to 4 weeks after the last engrafting of the human immunocompetent cells.

In a preferred embodiment, an infectious dose of 5x$10^2$ - $10^4$ $TCID_{50}$ for challenging of one HuSCID animal is used.

In a preferred embodiment, the efficacy of the HIV vaccine is determined by determining the viral load and / or the specificity an / or the intensity of an immune response in a body fluid sample of the mouse.

In a preferred embodiment, the body fluid sample is blood serum.

In a preferred embodiment, the inoculation with a vaccine is performed in the presence of an adjuvant.

In a preferred embodiment, HIV-specific immune response for PBMC-HuSCID or DC-PBMC-HuSCID mice is detectable within the period of several weeks after the last vaccination.

In a preferred embodiment, the evaluation of the vaccine is carried out by means of RT-PCR, Real Time PCR or ELISA.

## Figure Legends

Fig. 1 shows SCID mice immune deficiency testing compared to BalbC response for immunization with isolated U455 laboratory strain proteins.

Fig. 2 shows SCID-PBMC and BalbC immune response for immunization with cocktails of recombinant p120-1 and p120-2 proteins.

Fig. 3 shows PokA-79 infection rates on U937 and MT-4 background, 3 challenges 7 days b/w period.

Fig. 4 shows PokA-79 infection rates on U937 and MT-4 background, 2 challenges 3 days b/w period.

Fig. 5 shows PokA-79 - MT4 and U937-background's infection rates for different periods between challenging in SCID mice.

Fig. 6 shows PokA-79 strain in vitro replication dynamics, lymphocyte MT-4 and macrophage U937 cultures.

Fig. 7 shows PokA-79 strain in vitro replication dynamics, p24 expression, lymphocyte MT-4 and macrophage U937 cultures.

Fig. 8 shows Laboratory strains U455 and H9/IIIB infection rates on MT-4 and U937 cell cultures background, 4 challenges with 7 days b/w period.

Fig. 9 shows U455 and H9/IIIB in vitro replication dynamics on lymphocytes MT-4 and macrophage U937 backgrounds.

Fig. 10 shows U455 and H9/IIIB in vitro replication dynamics, p24 expression, lymphocyte MT-4 and macrophage U937 cultures.

Fig. 11 shows PokA-79 infection rates on cell culture U937 and MT4 background depending on the number of challenging.

Fig. 12 shows SCID-PBMC and SCID-cell culture background infection rates for challenging with PokA-79 laboratory strain.

Fig. 13 shows Recombinant p120-1 and p120-2 Immunization Effect for SCID-PBMC Challenging with PokA-79-MT-4 Laboratory Strain.

Fig. 14 shows Immune responses for p120-1 and p120-2 in challenged with PokA-79 - MT-4 laboratory strain SCID-PBMC Mice.

## Detailed Description

The present invention relates to the animal model for HIV vaccine effectiveness evaluation. In particular the presented method comprises endogenous immune system deficient CSID-humanized mouse model application for testing preventive HIV vaccine's immunization effect for in vivo HIV challenging prevention. SCID humanized peripheral blood lymphocytes (SCID-PBMC) or SCID humanized dendrite cells-engrafted mice (SCID-DC-PBMC) were used for immunizations and following challenging with HIV laboratory strains which envelop protein's pool was obtained according to reverse panning mass spectrometry analysis method. One donor PBMC or unmixed one donor DC-PBMC derivations were inoculated into one animal.

Severe Combined T- B- Immune Deficient (SCID) and thymus-free nude mice were never used in practice for in vivo evaluation of HIV vaccines effectiveness in spite it was known since early 90-ies that such mice can be successfully engrafted with human peripheral blood (PBMC), dendrite (DC) and stem cells capable for anti-HIV immune response formation [1, 13, 16] and in vivo HIV infection development [2, 3, 9, 12, 22]. An idea to combine these two features and to use HuSCID mice for vaccination assays was discussed [9]. Preclinical assays of DNA- and peptide-based HIV vaccine forms were run in a very limited number of animal models, mostly in two models only, i.e. in Rhesus Macaques and Chimpanzees (Chimps). In Rhesus Macaques human HIV infection does not work, so vaccines were tested exclusively for eliciting HIV-specific antibody responses detecting with extremely sensitive ELISPOT assays. In Chimps that are bearing HIV infection but without developing any symptoms of acquired immune deficiency syndrome (AIDS) finally test results were resumed to the same ELISPOT data. Therefore the existing monkey models are not at all satisfying since HIV infection does not at all develop in these animals like in humans. In general vaccines are distinguished from other therapeutic/preventive medical agents with their luck of the first stage of research in vitro in positive effect testing.

There are two main vaccine generations from beginning research in a vaccine development to finish with large scale medical purpose production. The first generation is represented by natural pathogen or its derivations deactivated with chemical compounds treatment and further neutralization of possible toxicity, fermentative pathogen's lysis into peptides and proteins, sterilization via autoclaving or any other similar method for vaccine active component's conservation. This way of vaccines creation was running for XXth century successfully for all bacterial pathogens like tuberculosis, plague, diphtheria, and also for large, stable DNA viruses diseases as smallpox, stable single- and double-strand RNA viruses diseases like morbilli, hepatitis A and B, human encephalitis, rabbies and other more rare tropical disease's vaccines. The first class of vaccines normally provides the formation of pathogen-specific, elongated and strong immune responses sufficient for infection development prevention. However the backwards of this vaccine's generation always were the lack of safety in administration due to high risk of survived pathogen's activity causing the development of real disease instead of immunization against it, and also anaphylactic reactions of immune system to foreign proteins and further immune vulnerability and disorders.

The second generation of vaccines development started with contributions of genetic engineering and molecular biology as recombinant proteins/peptides-based vaccines that are replacing the first class since the late 80-ies. Recombinant vaccines were quickly accepted since they provide a higher degree of safety in administration and production, since they are free of disease-evoking pathogens [14, 20]. In case they were targeted to conservative and stable pathogens after several years of efforts in development they matched highly specific immunity and close to complete protection against infectious disease. Recombinant proteins or peptide's mixtures produced in cheap and easy-to-maintain prokaryotic expression systems (E.coli transformed strains) are able to make exactly the mimic representation of prokaryote pathogen surface "checkpoints" crucial for antibodies recognition and vaccination against a number of bacterial infectious diseases. Bacterial cell surface normally contains a standard number of very conservative proteins, totally about one thousand. The similar situation with standard surface antigens exposition and their number is observed for many large DNA and RNA pathogenic viruses of different families like coronaviridae, adenoviridae, poxviridae, etc. Some small RNA viruses also have very stable genotype spread in its host practically all over the world - for example picomaviridae hepatitis A virus. For each known recombinant vaccine in history years were spent to solve the difficulty of distinguishing these protein's "checkpoints" responsible for immunity against the current pathogen from several hundred of possible candidates. Then immunogenic peptides cocktail preparation and pre-clinic efficacy assays took some more years of efforts. Within next several years of clinical trials doctors studied and demonstrated the advantages of recombinant vaccine over native pathogen's derivatives administration. This process normally took 15-50 years for every individual recombinant vaccine development.

If we try to transfer an HIV vaccine development attempts to this scheme of two stages described above it becomes obvious immediately that HIV vaccines do not fit there. There are several main reasons for it. First of all, the development of anti-HIV vaccine based on native pathogen described in several patents from early 90-ies [5, 11] was not possible due to impossibility to produce and collect the viral substrate in somehow reasonable amounts. In our research we certainly showed that it was not possible to concentrate virus envelop proteins with standard sucrose gradient untracentrifugation methods with over 200000g gravitation speed even in amounts sufficient for analytical purpose [7].

Therefore the first stage was absent for HIV vaccine, the second stage - recombinant vaccines development - were tried [8, 14]. The virus genome sequencing prior to attempts to create recombinant versions revealed so great variability of about one third viral genome that it was poor chances to find two identical full-length variants even from the same sample of one patient or group of patients mixed together [15]. We tried to estimate number of possible variations in main envelop protein gp120 for cohorts of patients applying new mathematics modeling based on differential analysis and molecular biochemistry parameters [23, 24]. The length of gp120 nucleotide sequence is variable and depends on deletions and insertions presence, their number and size. There is a scheme of gp120 mapping, where C1-C5 - conservative regions; V1-V5 - variable loops or variable regions.

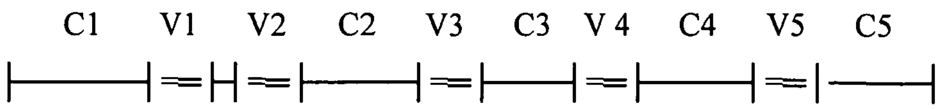


According to formula: $\ln N = \sum_{i,j} \alpha_{i,j} \ln C_{n_i}^{j}$ ,

where i - is number of variable nucleotide positions in env gp 120 sequence, $n_i$ - number of matching sites in each of i variable regions (in our case i=5, as variable regions of gp 120 are $V_1$, $V_2$, $V_3$, $V_4$, $V_5$), j - number of particles, that can be placed. In our case there are four particles - AT, GC and TA, CG.

According to experimental data total number of possible positions, in which nucleotides (A,T,G,C) can be allocated, is $N_{box} = N_{var} + N_{del}$ . Number of components - nucleotides, that will be allocated in boxes is $N_p = 4$ .

We can estimate variations number by calculating number of combinations by which can be realized states of these $N_p$ = 4 components in total number of boxes $N_{box}$:

$$C_{N_{box}}^{Np} = \frac{(N_{box})!}{(N_{box} - N_p)!(N_p)!} \quad [23]$$

Length of gp120 sequences used in our alignments is 1443 bp.

Alignment 1 - HIV-1 subtype A infected 30 individuals from Former Soviet Union territories and Africa. Nucleotide substitution positions $N_{var}$ = 546, there are deletions in 10 positions. Constant positions 887. Total number of positions = 1443. For calculation of possible variants number we must summarize number of nucleotide substitution positions and number of deletions. Altogether: $N_{box}$ = 546 + 10 = 556.

$$C_{556}^{4} = \frac{556!}{552!\bullet 4!} = 3939049415 \approx 3{,}9 \cdot 10^9$$

Alignment 2 - HIV-1 subtype A infected 10 individuals from Former Soviet Union territories. Nucleotide substitution positions $N_{var}$ = 331 , there are deletions in 30 positions. Constant positions 1082. Total number of positions = 1443. For calculation of possible variants number we must summarize number of nucleotide substitution positions and number of deletions. Altogether: $N_{box}$= 331 + 30 = 361.

$$C_{361}^{4} = \frac{361!}{357!\bullet 4!} = 695946630 \approx 6{,}9 \cdot 10^8$$

Researchers tried to overcome such genetic diversity for many years fishing out, cloning and reproducing fragments of the most conservative proteins or their domains. However it occurred that internal viral peptides like nef, pol and gag have no connection with virion's infectious activity and immunogenicity against it, and envelop proteins conservative fragments are small enough and are always covered with "loops" parts from outside of invading new host cells virions. These "loops" envelop peptides fragments were not only highly variable but extremely quickly mutation-altering regions at least up to now, until sequence information was obtained from standard infected individual's lymphocyte's DNA sequencing.

As a result now modem spectrum of HIV vaccines issued for clinical trials is represented with DNA vectors form - in terms of vaccines development evolution it is an incomplete recombinant second stage which did not yet reach a protein composition necessary for vaccination effect. So DNA HIV vaccine preclinical assays were run in Rhesus Macaques and Chimpanzees.

Meanwhile the animal model for studying human HIV infection and its treatment with antiviral agents was originally created in the late 80-ies by Derek Mosier [13] and improved by several laboratories in the world for different practical applications in HIV research [2, 6, 9, 10]. SCID mice engrafted with human leukocytes (PBL) provided human-like immune responses for immunization with different antigens including HIV peptides and proteins [1, 2, 4, 6]. Animals were supplied with 20-50x$10^6$ PBL intraperitoneally in the age of 2-5 weeks [9, 10], for the next two months they were examined for presence of human lymphocytes in peritoneal cavity, spleen, lymph nodes, liver and for human-like cytokine's profiles in bloodstream. After two-three months lymphocytes depletion, anemia, in some cases - leukemia development, were observed in grown up animals [9, 16]. To avoid such side effects (graft versus host disease - GVHD) it was offered to engraft immune deficient mice with dendrite cells produced via treatment of human leukocytes with human IL-2, IL-12, INF-$\gamma$ before engrafting [6, 9, 10]. Some researchers engrafted newborn CSID mice with parts of human organs and tissues - spleen, thymus, dendrite cells [19]. One human donor organ's tissue engrafting for a single mouse was successfully made by Stoddart group [18].

Different methods were adapted for modeling HIV infections in SCID and NOD-SCID mice, 95 percent of these experiments were based on laboratory HIV strains cultivated in vitro at cell cultures. CD4, CCR5 and CXCR4 receptors expressing cultures such as transfected HeLa-CD4, MT2, MT4, MAGI-CCR5, U937 and so on were used as an engrafted

substrate for viral infection [10, 21]. Grafts of human tissues (HuSCID Thy/Liv), isolated human leukocytes (PBL) and their infected with laboratory strain variants were administered intraperitoneally and provided HIV infection [2, 11, 22]. Infection rates were measured with RT-PCR or Real-Time PCR from intraperitoneal lavage, liver, spleen and thymus area [9]. But more often SCID mice are engrafted with dendrite cells cultivated in vitro with interleukin factors from human leukocytes (PBL). Such humanized chimerical mice are reported to be able to bear HIV infection for long periods of time (1-6 months) [3, 9] and were used for testing and demonstration of antiviral activity for different classes of antiretroviral agents [9, 17, 21]. It was demonstrated that engrafted with dendrite cells animals normally are protected from development of graft vs host disease and live for months after the xenograft embedding [22].

A number of researchers especially from those who was convinced in clinical trials HIV vaccines they developed themselves do not work proclaimed it is not possible to invent HIV immunization vaccine which will protect humankind from viral infection catching and development [17]. But it is not true because successful experiments with dendrite cells cultivated in vitro in presence of HIV laboratory strain cell culture or cultivating media and then engrafted into SCID or NOD-SCID mice showed these animals do not catch and do not maintain HIV infections from challenging with the same laboratory strain [9]. The same experiment with dendrite cells engraftment into SCID mice and blockage of infection with natural HIV isolates cultivated on healthy donors PBLs was successfully made once [9].

However in spite of high HIV-specific immunogeniety and ability to possess multiplying HIV infection nobody ever published results that SCID mice were used for testing DNA HIV vaccines efficacy [2, 6, 9, 10]. The question why HuSCID model was not exploited for vaccine research seems rather ritoric to us. This small animal model is matching all tasks for testing HIV immunization vaccines efficacy and much more appropriate for the purpose that macaque rhesus, rabbit, rat or dog models or even chimps. It is able to provide valid statistical analysis because little 15-25 gram of body weight mice need approximately 50 times less vaccine material for testing each than Macaques with several kilos body weight, and also it is a real possibility to provide mice with enough immunocompetent human cells material for engraftment.

The present invention demonstrates that huSCID mice represent an efficient animal model for the evaluation of an HIV vaccine.

Within the present invention, however, it is understood that also nude mice, immune deficient rats, immune deficient cats and other immunodeficient animals can be used.

Severe combined T-B-immune deficient mice (SCID mice) and methods for their production are known in the art. Furthermore, SCID mice are also commercially available.

Techniques for engrafting SCID mice with human immunocompetent cells are also known in the art and are, additionally, described in the attached example.

As used in the present invention the term "immunocompetent cells" comprises any cell of the specific immune system, such as lymphocytes and in particular T and B lymphocytes, which have been exposed to a particular antigen of interest, i.e. the HIV-vaccine to be evaluated, in vitro or in vivo. Examples for T-lymphocytes (T cells, thymus cells) are T-helper cells, cytotoxic T cells, NKT-cells, γδ-T cells, and memory T cells. T cells may be CD4 and/or CD8 positive and/or negative respectively, depending on their maturation status. Examples for B lymphocytes (B cells, bone cells), include plasma B-cells, B1- and B2-cells, marginal zone B cells and memory B cells. Immunocompetent cells may be obtained e.g. from individuals, such as peripheral blood mononuclear cells (PBMC), or from cell lines.

Preferably, the immunocompetent cells may be selected from the group consisting of PBMC, dendritic cells, B-cells, and T-cells. More preferably, the human immunocompetent cells are PBMC or dendritic cells.

Introduction of cells of human origin into the SCID mice may be achieved by using any conventional route, such as via the intraperitoneal or parenteral route. The intraperitoneal route is normally preferred due to the ease of administration and allowing administration of a volume of fluid. The cells may be introduced in form of a suspension in any suitable medium, allowing cell survival, such as phosphate buffered saline PBS or Dulbecco's modified Eagle's medium (DMEM), Roswell Park Memorial Institute medium (RPMI) or Glasgow's Minimal Essential Medium (GMEM).

The immunocompetent cells may be stimulated with immune stimulators prior to being introduced into the SCID mice. Immune stimulators for such purpose may be the immune stimulators mentioned above. Preferred stimulators include but are not limited to interleukin factors such as IL-2 (interleukin-2), IL-4 (interleukin-4), IL-12 (interleukin-12), and other factors such as INF-γ and GCSF (granulocyte colony stimulating factor). Further or different factors or combinations of such factors may also be used. The immune stimulators promote growth and/or development of e.g. T, B, and hematopoietic cells.

The dendritic cells may have been obtained by cultering human PBMC in the presence of cytokines capable of inducing the formation of dendritic cells in vitro. Methods for the production of dendritic cells in vivo are known in the art (see e.g. [25]).

The cell count for each introduction of cells of human origin may vary from about $1x10^3$ to $1x10^8$ cells per introduction, preferably from about $1x10^3$ to $1x10^7$ cells per introduction, more preferably from about $1x10^4$ to $1x10^7$ cells per introduction, even more preferred from about $1x10^5$ to $1x10^7$ cells per introduction, and even more preferred about $1\text{-}15x10^6$ cells per introduction (i.e. for a single engraftment only). The introduction of cells of human origin may be repeated prior to step (b), such as two to three times once weekly, or twice weekly, or providing a pause prior to a

third or further introductions.

In case PBMCs are introduced into SCID mice followed by immunization these mice are referred to as "PBMC-HuSCID mice". In case dendritic cells (DCs) are introduced into SCID mice followed by immunization and subsequent introduction of cells of the immunogenic background such as PBMCs, these mice are referred to as "DC-PBMC-HuSCID mice".

Preferably, the immunocompetent cells intended for one mouse are from one human donor. In case that various mice are used, it is preferred that for one assay, the imunocompetent cells are derived from one human donor and are then engrafted into the different mice.

As discussed above, the present invention relates to a method for the evaluation of an HIV vaccine, wherein a huSCID-mouse as defined above is inoculated with the HIV vaccine and thereafter challenged with HI-virus.

The vaccine may be applied/introduced in form of a suspension or mixture in any suitable medium and the exposure (vaccination) may be carried out in the presence of one or more adjuvants such as one or more pharmacological or immunological agents that modify the effect of the HIV-vaccine, such as immunopotentiators - also known as boosters of the immune system - which are effective in stimulating cell-mediated immunity. A preferred adjuvant for this purpose is Freund's adjuvant.

For an in vivo vaccination the vaccine to be tested may be administered directly to the mice on the day following the last introduction of immunocompetent cells or of the cells of the immunogenic background, or it may be administered some time (e.g. a couple of days or even weeks, preferably after two weeks) after the last introduction of said cells. Immunization may be carried out once or in a number of subsequent steps, e.g. it may be repeated after some time, e.g. after one to three weeks, preferably after two weeks two to four times, preferably three times.

The huSCID-mouse may be inoculated with the HIV vaccine 1 to 3 weeks after engrafting of the human immunocompetent cells.

Administering the vaccine to the mice may be performed using any route considered suitable, e.g. via the parenteral route or via the intraperitoneal route such as intravenous injection, subcutaneous injection, or intramuscular injection. The preferred route for administering the vaccine to the mice is via subcutaneous injection.

The vaccine to be tested may be administered to the HuSCID mice once, twice or more often (three to six times). Preferably, the HuSCID mice are vaccinated for a total of four times within two to four weeks, preferably within three weeks.

In case that the vaccine is a peptide or protein, the amount of vaccine peptides or proteins administered to the HuSCID mice may be between 1 and 100 μg, preferably between 10 and 80 μg, more preferably between 15 and 50 μg, even more preferably between 20 and 40 μg and most preferably about 25 μg of vaccine peptides or proteins in each step for each mouse. Administering may be performed in one or more individual steps, interrupted by lag phases lasting one day, days or weeks.

In case that the vaccine is a virus, the infection of the hu-SCID mouse be performed by using an amount of HIV strain infectious doses in cell culture, e.g. from about $5x10^2$ to $5x10^6$ $TCID_{50}$ (50% Tissue Culture Infective Dose), preferably from about $5x10^2$ to $5x10^4$ $TCID_{50}$. For example, for infecting HuSCID mice having freshly isolated PBMCs or DCs as background, from about $5x10^3$-$10^4$ $TCID_{50}$ of HIV-1 laboratory strain infectious doses in cell culture are administered to each individual mouse, or from about $5x10^2$ - $5x10^3$ $TCID_{50}$ infectious doses. Suitable cell cultures for this purpose include but are not limited to MT-4 or U937 culture cells. Preferably HuSCID mice can be challenged with $5x10^3$-$10^4$ $TCID_{50}$ of HIV-1 laboratory strain infectious doses in MT-4 culture cells, or with from about $5x10^2$ - $5x10^3$ $TCID_{50}$ infectious doses in U937 culture cells.

It will be understood that different ranges of cell numbers and $TCID_{50}$ may be used for infecting/exposing the HuSCID mice with/to HIV. Also, the HIV strains used for this purpose are not limited to a specific strain. Rather, all known HIV strains including HIV-1 strains and HIV-2 strains may be used as long as it is made sure that unprotected immunocompetent cells/mice (i.e. cells/mice not vaccinated with the HIV-vaccine to be tested) will be successfully infected with HIV. Preferably the HIV strain is an HIV-1 strain. More preferably the HIV strain is selected from the group consisting of U455, 9H/IIIB, and PokA-79.

In particular, an infectious dose of $5x10^2$ - $10^4$ $TCID_{50}$ for challenging of one HuSCID animal may be used.

As an alternative to challenging HuSCID mice with an amount of HIV strain infectious doses in cell culture, simultaneous challenging of HuSCID mice with a mixture of infected and uninfected cells may be performed. Such a mixture of infected and uninfected cells may comprise from about $1x10^4$ to $10x10^8$, preferably from about $1x10^5$ to $1x10^8$ and most preferably about $10x10^6$ of pure (uninfected) cells and from about $1x10^4$ to $10x10^7$, preferably from about $1x10^6$ to $1x10^7$ and most preferably about $5x10^6$ cells infected with an HIV strain. In a preferred embodiment SCID mice are challenged with e.g. 5-$10x10^6$ MT-4 or U937 pure culture cells and with $5x10^2$-$10^6$ $TCID_{50}$ of HIV-1 laboratory strain infectious doses in cell culture. Preferably the mice are challenged with $5x10^3$-$10^4$ $TCID_{50}$ of HIV-1 laboratory strain infectious doses in MT-4 culture, or with $5x10^2$ - $5x10^3$ $TCID_{50}$ infectious doses in U937 culture.

The challenges with HIV may be repeated for a number of times, e.g. for 2-5 times, preferably for 2 to 3 times with different time periods in between. A 3 day period between challenging is preferred for the achievement of the high

viral load numbers in a lymphocyte background culture and a 7 day period is preferred for a high viral load in a macrophage background culture.

For measuring the efficacy of a vaccine, all methods generally known in the art for that purpose may be used. In particular, the efficacy of the HIV vaccine may be determined by determining the viral load in a body fluid sample of the mouse. Said body fluid sample may be blood serum.

For determining the viral load every method known in the art can be used. This includes Real Time PCR, RT-PCR and ELISA.

The figures, scientific work and examples provided herein are intended to illustrate, but not to limit the invention.

Maintaining and experimental work with immune deficient animals are complicated with a number of special conditions and restrictions, however these expensive in vivo systems are worth the troubles they require for the survivorship. Thus, when new medicines are being tested for toxicity in vivo, SCID mice are known to deliver dosage regimes the most close to human's, while normal laboratory mice, rats and dogs $ID_{50}$ are 10-2 times higher and nude mice - 2-5 times lower than human ones. However to achieve informative results the researchers have to consider this number of conditions and adopt them for animals handling to avoid obtaining some false-positive data. The first little example of this approach is Fig. 1 presenting how SCID's immune status was checked before experiments with vaccines. SCID and BalbC mice were immunized with concentrated in 20% sucrose gradient ultracentrifugation U455 proteins mixture and ELISA results were compared with BalbC mice immune response. One of six SCIDs accidentally provided low immunity this time (Fig.1). In case SCID mice show some response for immunization (and it might happen for some percent of baby mice to be born with it) it is necessary to remove these animals from experiment assessment because viral infection in these mice might be blocked with this even low immunity and not with vaccination. For reproducing animals without such defect 3-5 cycles of brother-sister inbreeding is recommended.

4-5 weeks old SCID mice were inoculated with re-suspended in PBS (phosphate buffer saline) or DMEM without phenol red (Dulbecco's modified Eagle's media) 5-10x$10^6$ freshly isolated PBMC (standard Ficoll gradient total PBMC blood separation method) intraperitoneally once a week two times. Two weeks later the 3rd PBMC inoculation was performed and next day mice were immunized subcutaneously with peptides mixture obtained in two cycles of developed in lab technology [7] called p120-1 and p120-2. Each animal received 25 mkg peptides in cocktail mixed in 200 mkl incomplete Freund adjuvant for 1 hour at +4C. Immunization was repeated three times after two weeks, SCID mice were provided with fresh portions of PBMC weekly. The special requirement we provided was the supplying one donor PBMC 5-10x$10^6$ cells for each inoculation for one mouse (one donor's isolation was enough for a group of 5-10 animals). Immune response was tested in ELISA assays. One and two weeks after the last immunization SCID mice pre-treated with PBMC weekly had p120-1 and p120-2 immunity rates the same intensity with the response of normal BalbC mice (Fig. 1).

The other method of immune response formation in SCID mice we used included dendrite cells cultivation and initial mice supply. Donor PBMC were isolated with standard method and cultivated in vitro for 6 weeks in presence of differentiation blocking cytokine's factors - IL-2 (interleukin-2), IL-4 (interleukin-4) and GCSF (granulocyte colony stimulating factor) until cells are multiplied in number several times. 4-5 weeks SCID mice were inoculated with re-suspended in PBS or DMEM without phenol red 15x$10^6$ dendrite cells. Then after two weeks animals were immunized subcutaneously with p120-1 and p120-2 peptides mixture. Each animal received 25 mkg peptides in cocktail mixed in 200 mkl incomplete Freund adjuvant for 1 hour at +4C. Immunization was repeated three times after two weeks, SCID mice were supplied with fresh portions of PBMC once in 2-3 weeks. PBMC for next inoculations were isolated from the blood of the same donor whose blood was used as a source for dendrite cells cultivation for the current group of animals used in experiment. Response for immunization was the same in intensity as pre-treated with PBMC SCID mice showed but it didn't disappear completely after two weeks past since the last immunization (Fig. 2).

The conditions important for HIV-specific short-time immune boost are summarized in Table 1. There are relatively small number of PBMC - 5-10x$10^6$ cells for one animal for a single inoculation (1), the presence of PBMC (dendrite) cells substrate two weeks before every immunization (2), the implementation of one donor material for one mouse (group of mice) preventing them from GVHD development and early death (3). It is detectable also that intensity (specifity and values) of response after immunization/vaccination is 2-4 times higher in case one donor PBMC material is being used; immune response activity is comparable to that non-immune deficient mice elicit as murine antibodies production for the same immunization/vaccination (3). Several immunizations, preferably three, should be done during one vaccination course (4).

The quality of biomaterial used for vaccination is crucial for success of vaccinations. Immunogenic cocktails of recombinant HIV envelop peptides/proteins with sufficient not less than 20 mkg target peptide's concentrations for one animal should be used for successful vaccination (5). In case low-immunogenic and low-specific compositions such as HIV DNA vaccines are used HuSCID model will not provide positive results (6). It is preferable to use adjuvants for immune boost compositions in course of immunization/vaccination (Table 1). The limited period of detectable HIV-specific immune response (7-8) 3-4 weeks for PBMC and 5-6 weeks for DC-PBMC mice is due to: 1) animals were immunized subcutaneously with simple peptide's cocktails without immune boost composition; 2) small-animal models lifespan is

months, not years.

The composition/cocktail for successful immunizations against HIV challenging normally contains a great number of envelop sequences from decades for HIV laboratory strains to a library of hundreds and thousands for HIV-infected patient's isolates cloned as a pool in suitable expression system vector (See envelop variability analysis pp. 3-4).

**Table 1: HIV-specific immune boost conditions in PBMC-SCID and DC-PBMC-SCID mice.**

| ## | Applied Treatment (Conditions) for PBMC-HuSCID and DC-PBMC-HuSCID Mice Immunity for HIV env Peptides/Proteins | Immune Response for p120 when Treatment is: | |
|---|---|---|---|
| | | Applied | Not Applied |
| 1 | Modest PBMC, DC/PBMC number, 5-15x10$^6$ for one animal's each dose | + | - |
| 2 | Substrate (PBMC, DC) cells providing 1-2 weeks before immunizations | + | - |
| 3 | One donor PBMC material for each mouse during immunizations course | + | - |
| 4 | Three or more immunizations for the course with 2 weeks period before the next one for each PBMC-SCID, DC-PBMC-SCID mouse | + | - + |
| 5 | Vaccination/immunization with immunogenic HIV-env-specific components such as cocktails/mixtures of recombinant viral envelop peptides/proteins | + | - |
| 6 | Vaccination/immunization with HIV genes-containing viral vectors and non-viral plasmid DNA | - | - |
| 7 | Detectable env-specific immune boost time period for PBMC-HuSCID mice after the last immunization | 3-4 weeks | - |
| 8 | Detectable env-specific immune boost time period for DC-PBMC-HuSCID mice after the last immunization | 5-6 weeks | - |

Further experiments were targeted to test HIV laboratory strains challenging maintenance in HuSCID mice. The conditions, important for virus challenging formation on PBMC background in vivo are summarized in Table 2. There are two possible methods of infection modeling in SCID mice different in virus replication substrate:

1) donor PBMC or DC as background;
2) in vitro cultivation cell culture as background.

According to the first method 4-5 weeks old SCID mice were inoculated intraperitoneally with freshly isolated PBMC (standard Ficoll gradient total PBMC blood separation method) 5-10x10$^6$ one week before the next step. Before PBMC were rinsed twice from blood serum in PBS (phosphate buffer saline) with 10 min spinning at 1200rpm and then re-suspended in PBS or DMEM without phenol red. The next day after the second PBMC inoculation animals were inoculated intraperitoneally with log phase 5x10$^3$-10$^4$ $TCID_{50}$ one of HIV-1 laboratory strains infectious doses in cell culture MT-4 or 5x10$^2$ - 5x10$^3$ $TCID_{50}$ infectious doses in U937. Viral RNA titre in cell material used for challenging should be not less than 10$^{10}$ copies per ml for MT culture and 10$^8$ copies per ml for U937 culture; in vitro material controls were stored for Real Time PCR measurements. Challenges were repeated 2-3 times with different time periods between, SCID mice were provided with fresh portions of PBMC weekly. The special requirement was the supply one donor PBMC 5-10x10$^6$ cells for each inoculation for one HuSCID mouse (one donor's isolation was enough for a group of 5-10 animals). In case DC-PBMC were used for pre-challenging inoculations one donor freshly isolated PBMC were cultivated in vitro with presence of GCSF and IL-4 for 4-6 weeks as described above before inoculations into animals. Two 15-20x10$^6$ DC-PBMC cells inoculations with 2 weeks in-between period for one animal were necessary to maintain virus challenging.

According to the second method 5-8 weeks old SCID mice were inoculated intraperitoneally with 5-10x10$^6$ MT-

4 or U937 pure culture cells rinsed twice in PBS from cultivation serum with 10 min spinning at 800rpm and re-suspended in PBS. One week later pure cell culture inoculation was repeated. The next day after the second MT-4 or U937 cells inoculation animals were challenged intraperitoneally with $5x10^3$-$10^4$ $TCID_{50}$ one of HIV-1 laboratory strains infectious doses in cell culture MT-4 or $5x10^2$ - $5x10^3$ $TCID_{50}$ infectious doses in U937. Challenges were repeated 2-3 times with different time periods between, SCID mice were provided with fresh portions of PBMC weekly. The other way of challenging allows simultaneous inoculation of HuSCID mice with $10x10^6$ pure MT-4 or U937 culture and $5x10^6$ MT-4 or U937 infected with one of HIV-1 laboratory strains mixed in one syringe every 3-7 days, respectively. In both methods applied blood samples were collected from the animals 1-4 weeks after the last challenging and tested for viral infection rates by Real Time PCR, they achieved $10^3$-$10^8$ HIV RNA copies per ml in bloodstream.

In vivo virus modeling was performed with laboratory strains U455, 9H/IIIB and PokA-79 in our experiments. So infectious source was multiplied and prepared in log phase for inoculation either in MT-4(MT-2) or U937 culture. Immunity challenging blockage formation requires PBMC in vivo substrate, therefore we run both cell culture and PBMC for control challenging rates tests. The conditions for in vivo virus cultivation (see Table 2) are the presence of 5-15 million "fresh" or infection-free cell culture or PBMC intraperitoneally before challenging (1-2), minimal infectious dose supply for each animal (3), continuous challenging detection period for one donor PBMC material for each animal's treatment (4-7) and matching typical for cell's background in vivo virus cultivation dynamics (8).

Two more consequences are important for modeling HIV challenging in HuSCID mice:

1) Virus strain/isolate multiplication kinetics and animal's challenging timing;
2) The difference between laboratory strain infective activity for cell culture and PBMC substrates.

**Table 2: HIV challenging formation conditions in PBMC-, DC-PBMC-SCID mice.**

| ## | Applied Treatment (Conditions) for PBMC-HuSCID and DC-PBMC-HuSCID Mice HIV Infection Rates in Bloodstream | Detectable in Bloodstream HIV RNA Copies/ml Load When Treatment is: | |
|---|---|---|---|
| | | Applied | Not Applied |
| 1 | Modest PBMC, DC/PBMC number, $5-15x10^6$ for one animal | + | - |
| 2 | Substrate (PBMC, DC, cell culture) cells provided within one week before challenging | + | - |
| 3 | $2-5xl0^6$ cells minimal infectious dose for challenging of one SCID animal | + | - |
| 4 | One donor PBMC material for each animal | months | 2 weeks |
| 5 | Two or more infected cells inoculations for every animal with 2 weeks period between inoculations, viral RNA copies/ml | $10^4$-$10^8$ | 0-$10^3$ |
| 6 | Detectable viral load time period for PBMC-HuSCID mice after the challenging | 1-2 weeks | - |
| 7 | Detectable viral load time period for DC-PBMC-HuSCID mice after the challenging | 2-4 weeks | - |
| 8 | Virus cultivation dynamics typical for cell's background in vivo | + | - |

HIV laboratory strains can have aggressive or slow multiplication kinetics; quickly developing strains are better and more demonstrative for in vivo experiments. We have used only highly aggressive strains of A and B subtypes with fast replication dynamics and high rates of viral concentration in vitro - $10^{10}$- $10^{12}$ copies/ml HIV RNA within 1-4 days of cultivation (Table 3). But cell culture virus replication kinetics has the same value for in vivo viral load and percentage of successful challenging. Analysis of viral load in SCID-PBMC mice bloodstream after 2-4 times challenging with the same laboratory strain cultivated on different cell cultures shows dependency of HIV RNA copies rate from the regime of inoculation (Fig. 3-5). When inoculations of infected with laboratory virus cells were made 3 days after the previous ones rates of viral load (HIV RNA copies per one ml of mice blood number) were always several times higher for variant which was cultivated on MT-4 culture background (Fig.3) than for the variant cultivated on macrophage's U937 background. When inoculations were made 7 days after the previous ones HIV RNA copies numbers were always several times higher for cultivated on U937 background variant (Fig.4) than for the variant cultivated on lymphocyte's MT-4 background. In one experiment viral load in PBMC-SCID bloodstream was also 3-10 times higher for MT-4 cultivation with 3 days period between challenging than for U937 cultivation and 3-15 times higher for U937 cultivation with 7 days period between challenging than for MT-4 cultivation (Fig. 5). The same in vivo HIV infection development dynamics has been observed for U455 and H9/IIIB laboratory strains (Fig. 8).

**Table 3: Titre and dynamics of HIV laboratory strains viral replication on lymphocyte-type and macrophage-type cultivation cell cultures, p24 and RNA copies/ml**

| HIV Lab. Strain | Cell Culture | Time of Incubation Period, hours | | | | | | | | | | | |
|---|---|---|---|---|---|---|---|---|---|---|---|---|---|
| | | 0 | | 24 | | 48 | | 72 | | 96 | | 120 | |
| | | p24, P/N | RNA, c/ml | p24, P/N | RNA, c/ml | p24, P/N | RNA, c/ml | p24, P/N | RNA, c/ml | p24, P/N | RNA, c/ml | p24, P/N | RNA, c/ml |
| **U455** | **MT-2** | 2.3 | 4.8x10$^{8}$ | 18.1 | 3.3x10$^{11}$ | 22.5 | 5.2x10$^{11}$ | 14.4 | 3.4x10$^{11}$ | 14.1 | 9.5x10$^{10}$ | 8.8 | 9.2x10$^{8}$ |
| | **MT-4** | 2.7 | 2.4x10$^{8}$ | 17.6 | 8.9x10" | 23.3 | 7.4x10$^{11}$ | 22.2 | 8.1x10$^{11}$ | 21.3 | 3.6x10$^{11}$ | 17.4 | 8.4x10$^{9}$ |
| | **U937** | 2.4 | 1.6x10' | 18.3 | 3.9x10$^{9}$ | 20.7 | 2.9x10$^{10}$ | 20.4 | 7.7x10$^{10}$ | 19.1 | 5.6x10$^{10}$ | 17.4 | 2.5x10$^{8}$ |
| **H9/IIIB** | **MT-2** | 3.0 | 5.9x10$^{7}$ | 20.7 | 5.7x10$^{12}$ | 22.1 | 3.8x10$^{12}$ | 22.3 | NM* | 22.4 | NM* | 13.0 | NM* |
| | **MT-4** | 2.4 | 3.5x10$^{8}$ | 21.8 | 4.5x10$^{12}$ | 20.9 | 2.7x10$^{12}$ | NM* | NM* | NM* | NM* | NM* | NM* |
| | **U937** | 2.7 | 6.1x10$^{7}$ | 19.3 | 2.9x10$^{10}$ | 22.4 | 7.8x10$^{9}$ | 25.7 | 8.5x10$^{10}$ | 25.4 | 5.1x10$^{11}$ | 18.5 | 1.7x10$^{9}$ |
| **PokA-79** | **MT-2** | 2.0 | 4.2x10$^{8}$ | 16.2 | 8.2x10$^{12}$ | 20.1 | 2.3x10$^{13}$ | 18.1 | 2.7x10$^{12}$ | 16.3 | 1.9x10$^{10}$ | 8.0 | NM* |
| | **MT-4** | 2.9 | 5.4x10$^{8}$ | 17.3 | 8.3x10$^{10}$ | 20.0 | 9.2x10$^{10}$ | 26.0 | 6.4x10$^{9}$ | 26.1 | 7.3x10$^{9}$ | 16.0 | 7.9x10$^{8}$ |
| | **U937** | 2.8 | 8.6x10$^{7}$ | 23.0 | 1.8x10$^{9}$ | 25.2 | 5.5x10$^{9}$ | 28.2 | 6.6x10$^{9}$ | 29.1 | 9.8x10$^{9}$ | 27.5 | 2.5x10$^{9}$ |
| NM* - not measured | | | | | | | | | | | | | |

In vivo HIV-1 laboratory strains cultivation dynamics on lymphocyte's and macrophage's background repeats precisely in vitro dynamics of virus strains multiplication. So, in vitro replication titre for U455, PokA-79 and H9/IIIB measured both as Real Time HIV RNA copies data and p24 expression ELISA results were reaching maximums in 24-48 hours for lymphocyte MT-4 and MT-2 cell cultures (Table 3, Figs 6-7, 9). Contrariwise, in vitro replication titre for U455, PokA-79 and H9/IIIB measured both as RNA copies and p24 expression were growing to maximums within 72-96 hours for macrophage U937 cell culture (Table 3, Figs 6-7, 9). As it is necessary to use log phase infected cell culture for in vivo challenging success we can conclude 3 days period between challenging is the best for the achievement of the highest viral load numbers in lymphocyte culture background and 7 days period - for the highest viral load in macrophage culture background.

The activity of in vivo challenging (bloodstream viral load) and in vitro infection titre are very different for HIV laboratory strains multiplying on different backgrounds. In vivo challenging rates (Fig. 12, Table 4) and in vitro multiplication dynamics (Fig. 6, Table 5) were analyzed for PokA-79 laboratory strain on freshly isolated human peripheral blood mononuclear cells (PBMC) and cell cultures background. In vivo PokA-79 challenging rates on PBMC background were not detectable as HIV RNA copies in animals bloodstream after one single infection's inoculation, achieved 10$^{3}$ viral RNA copies per ml after two inoculations and 10$^{3}$-10$^{4}$ viral RNA copies per ml after three infected material inoculations (Table 4, 3$^{rd}$ column). PokA-79 strain challenging rates for lymphocyte MT-4 in vivo infection modeling were 10$^{2}$-10$^{3}$ RNA copies per ml after the first inoculation, 10$^{3}$-10$^{5}$ - after two inoculations and 10$^{4}$-10$^{6}$ HIV RNA copies per ml in bloodstream after three infected material challenges (Table 4, 1$^{st}$ column). PokA-79 challenging rates for macrophage U937 in vivo infection modeling were 10$^{3}$ RNA copies per ml after the first inoculation, 10$^{3}$-10$^{5}$ - after two inoculations and 10$^{5}$-10$^{7}$ HIV RNA copies per ml in bloodstream after three infected material challenges (Table 4, 2$^{nd}$ column). These rates on PBMC background in average were 10$^{2}$-10$^{4}$ times lower for two and three challenging than the same results obtained for cell culture background (Fig. 12, Table 4). The infection regimes for PBMC background were similar to macrophage-based modeling, so challenging viral RNA rates were detectable in SCID-PBMC mice bloodstream after two-three inoculations when period between challenges was 7 days (Table 4 data). If 3 days period was settled between challenges in SCID-PBMC mice like it is being done for higher rates in lymphocyte-background SCID HIV challenging modeling the results in animals bloodstream were not detectable in 45 percent of taken samples.

**Table 4: PokA-79 laboratory strain challenging rates on PBMC and cell cultures background in vivo.**

| Animal Groups | PokA-79 Challenging Viral Load, RNA copies/ml | | | | | | | | |
|---|---|---|---|---|---|---|---|---|---|
| | MT-4 | | | U937 | | | Human PBMC | | |
| | I | II | III | I | II | III | I | II | III |
| 1 | 9.9x10$^{2}$ | 6.9x10$^{5}$ | 1.6x10$^{5}$ | 6.0x10$^{3}$ | 9.9x10$^{3}$ | 5.2x10$^{6}$ | - | 5x10$^{2}$ | 7.5x10$^{3}$ |
| 2 | 2.0x10$^{3}$ | 3.9x10$^{4}$ | 9.8x10$^{6}$ | 5.7x10$^{3}$ | 3.0x10$^{5}$ | 1.2x10' | - | 5.2x10$^{3}$ | 2.8x10$^{4}$ |
| 3 | 3.9x10$^{3}$ | 3.9x10$^{4}$ | 2.7x10$^{4}$ | 2.2x10$^{3}$ | 3.6x10$^{4}$ | 4.8x10$^{5}$ | - | - | 4.0x10$^{4}$ |
| 4 | - | 8.4x10$^{3}$ | 2.6x10$^{5}$ | - | 3.0x10$^{4}$ | 8.6x10$^{5}$ | - | 8.6x10$^{3}$ | 9.0x10$^{3}$ |

We supposed the lower rates for PBMC virus challenging background to be surprising and not expectable, therefore we studied it's correlation with in vitro dynamics. In vitro cultivation titres of virus strain on PBMC background were always lower 10$^{2}$-10$^{5}$ times than on competent for it's cultivation cell cultures background; it was true for both HIV RNA copies per ml Real Time detection and for p24 expression ELISA calibration methods. So, lymphocyte-based PokA-79 cultivation on MT-2 and MT-4 cell lines brings titres to 10$^{11}$- 10$^{13}$, macrophage-based cultivation on U937 cells - to 10$^{9}$ copies/ml RNA, and cultivation on human PBMC - to 10$^{7}$- 10$^{8}$ RNA copies/ml only (Table 5, Fig. 6, the numbers of infected and fresh cells in cultures were standardized to equal ones). In vitro dynamics of laboratory strain multiplication for human PBMC background is similar to macrophage cell culture background: infection titres grow slowly and achieve maximums at 3$^{rd}$-5$^{th}$ day of cultivation (Fig. 6), whenever lymphocyte-based cultivation brings HIV RNA and p24 titres hit maximums at 1$^{st}$-2$^{nd}$ day of cultivation and at 3$^{rd}$ day titres of HIV RNA copies start to go down (Table 5). However macrophage-based in vitro cultivation viral RNA titres start to drop down after 5$^{th}$ day of cultivation unlike PBMC-based model titres that continue approximately the same levels for 10 days of cultivation (Table 5) and even longer up to 3 weeks.

In vitro PokA-79 laboratory strain viral replication on PBMC and cell cultures background analysis explains in vivo inoculation regimes and challenging rates for SCID-PBMC HIV infection modeling but it does not reveal the reason why these HIV titre numbers are so very much different for PBMC and cell cultures. Our experience with mass spectrometry analysis of HIV-1 envelop protein variability run for laboratory strains PokA-79, U455 and H9/IIIB and for virus isolates from several cohorts of HIV-1 subtype A infected individuals RF territory-inhabitants [6] brought the only possible conclusion. Laboratory strains selected in cultivation on one or two cell cultures expressing CD4 and/or one of co-receptors CCR5 or

**Table 5: PokA-79 laboratory strain viral replication on PBMC and cell cultures background in vitro.**

| Culture background | Time of Incubation Period, hours | | | | | | | |
|---|---|---|---|---|---|---|---|---|
| | 0 | 24 | 48 | 72 | 96 | 120 | 144 | 240 |
| | PokA-79 RNA copies in 1 ml cell suspension in culture media | | | | | | | |
| **PBMC** | $7.0x10^7$ | $6.5x10^7$ | $1.8x10^8$ | $9.6x10^7$ | $3.8x10^8$ | $4.8x10^7$ | $8.7x10^7$ | $7.8x10^8$ |
| **MT-2** | $7.0x10^7$ | $7.5x10^{12}$ | $5.4x10^{12}$ | $7.9x10^{11}$ | $7.2x10^{10}$ | $8.2x10^8$ | NM* | NM* |
| **MT-4** | $7.0x10^7$ | $8.2x10^{12}$ | $2.3x10^{13}$ | $2.7x10^{12}$ | $1.9x10^{10}$ | $7.9x10^8$ | NM* | NM* |
| **U937** | $8.6x10^7$ | $1.8x10^9$ | $5.5x10^9$ | $6.6x10^9$ | $9.8x10^9$ | $2.5x10^9$ | NM* | NM* |

CXCR4 in the way how it is normally represented on monoclonal cell culture surface have luck of gp120/gp160 envelop protein's variations. Cell cultures are characterized as monoclonal originated population of human tissue material selected in cultivation in vitro in permanent conditions. Due to monoclonal and constant conditions cultivation cell cultures represent relatively small number of variations of CD4 (CCR5, CXCR4) receptors available for recognition and infection compared to human cells in the bloodstream, PBMC. Therefore the variability of gp120/gp160 for HIV laboratory strains is hundreds or thousands times restricted versus native virus envelop proteins. It means if laboratory strain was put into SCID-PBMC environment in vivo or donor PBMC isolates in vitro it has to invade human PBMC instead of cell culture for it\s further multiplication. This viral strain survives with lower titre of infectivity and slower infection development kinetics (Tables 4-5, Figs 6, 12) because it's existing gp120/gp160 modifications can recognize only small number of existing variations of CD4/co-receptors on PBMC surface.

$TCID_{50}$ (tissue cell infectivity dose) is one of the crucial parameters of in vivo HIV challenging because viral load (viral RNA copies number in blood serum or tissue) and virus infective potential are different values in calculation for the same virus particles number. $TCID_{50}$ is a parameter that characterizes alive and active for new cells invasion virus units. Even in log phase laboratory strain in vitro culture $TCID_{50}$ referred to the number of HIV viral particles is minimum 100-1000 times lower than total viral particles number, and in overgrown viral in vitro culture where cell deaths are closer to 95-100 percent the difference between $TCID_{50}$ and RealTime PCR data is $10^3$-$10^7$ in viral particles representation. A simple correlation between viral activity measured in $TCID_{50}$ and HIV preventive vaccine effectiveness is like this: the higher is $TCID_{50}$, the lower are chances that HIV-specific immunity can block further challenging effectively. However in reality HIV infection doses transmitted from human to human during sexual contact or IDU (intravenous drug usage) are modest, incomparably lower than ones we run in animal's challenging. Therefore the possibility of HIV infection blockage with specific immune-boost vaccination can be applied into practice.

Infectious doses calculation (Mensch-Reed protocol) in $TCID_{50}$ units was performed via gradual dilutions method and measured within a period of three weeks in ELISA tests as p24 expression. The infectious dose is correspondent to amount of newly replicated and secreted into cultural media infective virus. All samples were taken from infected cell culture before concentration of material for every animal's challenging and evaluated as average for two similar experiments. $TCID_{50}$ data for three laboratory strains are presented in table 6. 3rd and 4th columns contain $TCID_{50}$ data in 1 ml culture suspension for all three laboratory strains on MT-4, MT-2 and U937 cell culture background for 24 and 48 hours of incubation, respectively. 5th column shows $TCID_{50}$ which was applied to one single animal every inoculation calculated as measured for 24 hours log phase incubation multiplied to 10 ml infected cell culture suspension in average used for concentration of infectious material for one mouse challenging.

$TCID_{50}$ 24 hours (3rd column) for any of HIV-1 laboratory strains on MT-4 cell culture background varied within $5x10^3$-$10^4$ units/ml culture media, $TCID_{50}$ 48 hours (4th column) - within $6.8x10^6$ - $9.6x10^7$ units/ml. $TCID_{50}$ 24 hours (3rd and 4th columns) on U937 cell culture background was $7x10^2$ and for 48 hours -$3x10^3$ -$8x10^3$ only. The dynamics of $TCID_{50}$ increase was the same for U455, H9/IIIB and PokA-79 laboratory strains. These data show that there is a great difference in $TCID_{50}$ growth between 24 and 48 hours of incubation for lymphocyte cell culture background infection but not much difference in $TCID_{50}$ growth between 24 and 48 hours of incubation for macrophage U937 cell culture background. It means that maximum $TCID_{50}$ infectious doses for macrophage U937 laboratory strains HuSCID mice challenging could be achieved after 48-72 hours of culture in vitro incubation before challenging. So we started to run 48 hours incubation period for U937-based HIV strains challenging and achieved higher levels of RNA copies viral load in animal's bloodstream.

**Table 6: Tissue Culture Infectious Doses ($TCID_{50}$) used for HuSCID challenging.**

| Laboratory Strain | Infected Culture background, up to $10^7$/animal | $TCID_{50}$ in 1 ml 24 h | $TCID_{50}$ in 1 ml 48 h | TCID50 for Each HuSCID Animal, 24 h |
|---|---|---|---|---|
| U 455 | MT-4 | $1.8x10^3$ | $2.9\ x10^7$ | $18x10^3$ |
| | MT-2 | $3.5x10^3$ | $7.2x10^7$ | - |
| | U937 | $6.9x10^2$ | $5.4x10^3$ | $6.9x10^3$ |
| H9/IIIB | MT-4 | $8.7x10^3$ | $4.1\ x10^7$ | $87x10^3$ |
| | MT-2 | $4.7x10^4$ | $9.6\ x\ 10^7$ | - |
| | U937 | $7.2\ x10^2$ | $8.2x10^3$ | $7.2x10^3$ |
| PokA-79 | MT-4 | $1.9x10^3$ | $6.8x10^6$ | $19x10^3$ |
| | MT-2 | $4.3\ x10^3$ | $2.9\ x10^7$ | - |
| | U937 | $7.1\ x10^2$ | $3.3x10^3$ | $7.1x10^3$ |

## Examples

**Example 1.** Vaccination with PokA-79 strain envelop protein cocktails and challenging with PokA-79 in SCID-PBMC mice.

Immunization effect tests were completed for laboratory strain PokA-79. The application of principle of mass spectrometry analysis of represented in majority HIV-1 gp120/gp160 epitopes for vaccine development brought the material for SCID-PBMC immunizations. These immunizations occurred to be able to prevent or suppress further animal's challenging with the same laboratory HIV strain which aminoacid sequences recognitions were used for design of primers for major gp120/gp160 epitope's cloning and expression.

The groups of animals were treated as follows: 4-5 weeks old SCID mice were inoculated with 5-$10x10^6$ freshly isolated donor PBMC intraperitoneally once a week or with DC-PBMC once in three weeks. Two weeks later the 1st PBMC inoculation or the 1st DC-PBMC grafting mice were divided into groups (3 mice in every group) and immunized subcutaneously with peptides mixture p120-1 or p120-2. Each animal received 25 mkg peptides in cocktail mixed in 200 mkl incomplete Freund adjuvant for 1 hour at +4C. Immunization was repeated three times with the between period of two weeks. Immune response was tested in ELISA assays (Fig. 14). Three days after the last immunization and the scheduled PBMC inoculation animals were challenged intraperitoneally with log phase $5x10^{6-}$ $10x10^6$ MT-4 culture infected with PokA-79. Viral RNA titre in cell material used for challenging should be not less than $10^{10}$ copies per ml for MT-4 culture; in vitro material controls were stored for Real Time PCR measurements. Control HuSCID mice were challenged intraperitoneally with the same amount of MT-4 culture infected with PokA-79 in the same time with experimental groups without previous immunizations. Challenges were completed 2 times with three days periods between, HuSCID mice were provided with fresh portions of PBMC weekly or with DC-PBMC once in three weeks. Animals were sacrificed one week after the second challenging and blood serum samples were sent for ELISA and Real Time PCR measurements.

There were three groups of SCID-PBMC immunized with p120-1 and four groups immunized with p120-2; serums of BalbC mice immunized with p120-1 and p120-2 were taken as controls for immunization results detection (Fig.14). In mice serums diluted 4-14 times with quantitative calibration curve p120-1 and p120-2 antibodies titre were detectable for all SCID-PBMC animals, however control BalbC mice HIV env-specific antibodies titre were always higher (approximately 2-2.5 times in ELISA test) than SCID-PBMC animals titre (Fig.14). p120-1 and p120-2 immune responses assessment was positive for immunized SCID-PBMC mice for 3 weeks after the last immunization and for 2 weeks after the last immunization for BalbC mice (Fig. 2). HIV titre in bloodstream measured with Real Time PCR was not detectable in 4 groups immunized with p120-2. In one of 3 groups immunized with p120-1 two animals provided challenging rate 400 and 500 HIV RNA copies/ml (Fig. 13). SCID-PBMC mice challenged with PokA-79 - MT-4 strain without immunization pre-treatment were tested as positive controls (Fig. 13). Positive PokA-79 viral RNA titre in bloodstream of one of 3 SCID-PBMC - p120-1 groups (Fig. 13) correlated with the lowest ELISA p120-1 antibodies titre evaluated for the same SCID-PBMC-p120-1 animal's group (Fig. 14).

## References

1. Bankert RB, Chen Fang-An "SCID Mouse Having a Normal Immune Response to Exogenous Antigens" Patent EP0937389 (A2), C12N5/00; A01K67/027, 1999-08-25;

2. Bonyhadi ML, Kaneshima H. "The HuSCID Mouse: an in vivo Model for HIV Infection in Humans" Molecular medicine today, 1997 June, pp 246-253;

3. Brainard D.M, Seung E., Frahm N., Cariappa A., Bailey C.C. et al. "Induction of Robust Cellular and Humoral Virus-Specific Adaptive Immune Responses in Human Immunodeficiency Virus-Infected Humanized BLT Mice", J. Virology, 2009 July; 83(14): 7305-7321;

4. Brams P. Morrow PR "Neutralizing high affinity human monoclonal antibodies specific to RSV F-protein and methods for their manufacture and therapeutic use thereof' Patent US2002001798 (A1), C07K16/00; C07K16/10, 2002-01-03;

5. Capodici J. et al., "Large-Scale Beta-Propiolactone Inactivation of HIV for Vaccines" Bioprocess Int., 2006, Feb., pp 36-41;

6. Coccia MA, Brams P. "Methods for Producing Human Antibodies in SCID Mice Using Dendrite Cells" WO Patent #WO9912553 (A1), C07K16/40, A61K39/00; 1999-03-18;

7. Filinova E. «HIV preventive vaccine based on HIV specific antibodies» Patent WO2009046984 (A1), US20070978536P 20071009, C12N15/10, A61K39/21, 2009-04-16;

8. Franchini G., Hel Z., Pavlakis G. et al. "Improved immunogenicity using a combination of DNA and Vaccinia virus vaccines" Appl.: The Government of USA, A61K 39/21 (2006.01), WO/2001/082964, 08.11.2001;

9. Friedman H., Specter S., Bendinelli M. "In vivo Models of HIV Disease and Control" Springer, 2006, Chapter 2: Anderson E.R. at al. "Animal Model Systems of HIV-Diseases"; Chapter 4: Laperanta K. et al. "SCID Mice Transplanted with Human Cells as Small Animal Models in AIDS Research";

10. Ito M., Kobayashi K., Nakahata T. et al. "Method of producing a mouse suitable for engraftment, differentiation and proliferation of heterologous cells, mouse produced by this method and use of the mouse" Patent US2007011753 (A1), 2007-01-11;

11. Karp N.M. "Immunogenic Composition and Method of Developing a Vaccine Based on Psoralen Inactivated HIV" Patent WO2005040353, 2005-05-06;

12. Lapenta C, Fais S, Rizza P, Spada M, Logozzi MA et al. "U937-SCID mouse xenografts: a new model for acute in vivo HIV-1 infection suitable to test antiviral strategies" Antiviral Research 36 (1997) 81-90;

13. Mosier DE, Gulizia RJ, Baird SM, Wilson DB. "Transfer of a functional human immune system to mice with severe combined immunodeficiency" Nature, 1988 Sep 15;335(6187):256-9;

14. Picker, L. J., Jarvis, M., Nelson, J, A. "SIV and HIV vaccination using RHCMV- and HCMV-based vaccine vectors" US Patent C12N 15/63 (2006.01), Appl.: Oregon Health and Science University, WO/2006/031264, 23.03.2006;

15. Poignard P. et. al., "Heterogeneity of envelope molecules expressed on primary human immunodeficiency virus type 1 particles as probed by the binding of neutralizing and non-neutralizing antibodies" J. Viro1.2003.Jan.;77.(1.): 353-65;

16. Sehon A, Bitoh S. "Testing Individual Immune Response to an Antibody with SCID Mouse" Patent WO9405779 (A1), A01K67/027; A01K67/027; 1994-03-17;

17. Steinbrook R. "One Step Forward, Two Steps Back - Will There Ever Be an AIDS Vaccine?" New England Journal of Medicine, Dec. 27, 2007(26), 357:2653-2655;

18. Stoddart C.A, Bales C.A, Bare J.C, Chkhenkeli G., Galkina S.A. et al. "Validation of the HuSCID Thy/Liv Mouse Model with Four Classes of Licensed Antiretrovirals", PLoS August 2007 Issue 8 p655;

19. Su L., Kaneshima H. Bonyhadi M., Salimi S., Kraft D., Rabin L., and McCune J. M. "HIV-1 -Induced Thymocyte Depletion Is Associated with Indirect Cytopathicity and Infection of Progenitor dells In Vivo", Immunity, Jan. 1995, V. 2, pp 25-36;

20. Uckun FM "Pokeweed Antiviral Protein Polypeptides with Antiviral Activity" Patent WO03106479 (A2), C12N15/09; A61K38/00, 2003-12-24;

21. William C. Griffin WC, Middaugh LD, Cook JE, Tyor WR "The severe combined immunodeficient (SCID) mouse model of human immunodeficiency virus encephalitis: Deficits in cognitive function" Journal of Neurovirology, Volume 10, Issue 2 April 2004, pp 109-115;

22. Van Duyne R, Pedati C, Guendel I., Carpio L, Kehn-Hall K, Saifuddin M, Kashanchi F. "The utilization of humanized mouse models for the study of human retroviral infections" Retrovirology 2009, 6:76;

23. Fikhtengholtz G.M. "Differential and Integral Math Analysis", 7th edition, volume2, Moscow, 1969, *in Russsian;

24. Wolkenstein M.V. "Molecular Biophysics", Moscow, "Nauka", 1975, * in Russian.

25. Jonuleit H, Kuhn U, Muller G, Steinbrink K, Paragnik L, Schmitt E, Knop J, Enk AH.: Pro-inflammatory cytokines and prostaglandins induce maturation of potent immunostimulatory dendritic cells under fetal calf serum-free conditions. Eur J Immunol. 1997 Dec; 27(12):3135-42.

**Claims**

1. The use of a Severe Combined T-B-Immune Deficient (SCID) mouse engrafted with human immunocompetent cells (huSCID-mouse) as an animal model for the evaluation of the effectiveness of an HIV vaccine.

2. The use of claim 1, wherein the immunocompetent cells are able to develop a human-type immune reaction for HIV.

3. The use of claim 1, wherein the human immunocompetent cells are PBMC.

4. The use of claim 1, wherein the human immunocompetent cells are dendritic cells.

5. The use of claim 1, wherein the immunocompetent cells are a mixture of PBMC and dendritic cells.

6. The use of claim 5, wherein the dendritic cells have been obtained by cultering human PBMC in the presence of cytokines capable of inducing the formation of dendritic cells in vitro.

7. The use of any of claims 1 to 6, wherein the human immunocompetent cells are derived from one human donor.

8. The use of any of claims 1 to 7, wherein the mouse has been engrafted with 5-15x$10^6$ cells.

9. The use of any of claims 1 to 8, wherein the huSCIDmouse is the particular breed of animals deficient in their own endogenous immune system and introduced with human immunocompetent cells or any other human cells.

10. A method for the evaluation of an HIV vaccine, wherein a huSCID-mouse as defined in any of claims 1 to 9 is inoculated with the HIV vaccine and thereafter challenged with HI-virus.

11. The method of claim 10, wherein the HIV vaccine is an HIV-1 envelop peptides/proteins cocktail, preferably wherein the proteins or peptides are of recombinant origin.

12. The method of any of claims 10 or 11, wherein the evaluation of the vaccine is determined by determining the efficacy of the vaccine.

13. The method of claim 12, wherein the efficacy is determined by determining the protection to HIV challenge.

14. The method of any of claims 10 to 13, wherein the huSCID-mouse is inoculated with the HIV vaccine 1 to 4 weeks after the last engrafting of the human immunocompetent cells.

15. The method of any of claims 10 to 14, wherein an infectious dose of 5x$10^2$ - $10^4$ $TCID_{50}$ for challenging of one HuSCID animal is used.

16. The method of any of claims 10 to 15, wherein the efficacy of the HIV vaccine is determined by determining the viral load and / or the specificity an / or the intensity of an immune response in a body fluid sample of the mouse.

17. The method of claim 16, wherein the body fluid sample is blood serum.

18. The method of any of claims 10 to 17, wherein the inoculation with a vaccine is performed in the presence of an adjuvant.

19. The method of any of claims 10 to 18, wherein HIV-specific immune response is detectable within the period of several weeks after the last vaccination.

20. The method of any of claims 10 to 19, wherein the evaluation of the vaccine is carried out by means of RT-PCR, Real Time PCR or ELISA.

**Figure 1: SCID Mice Immune Deficiency Compared to BalbC Immune Response for Isolated U455 Lab. Strain Proteins**

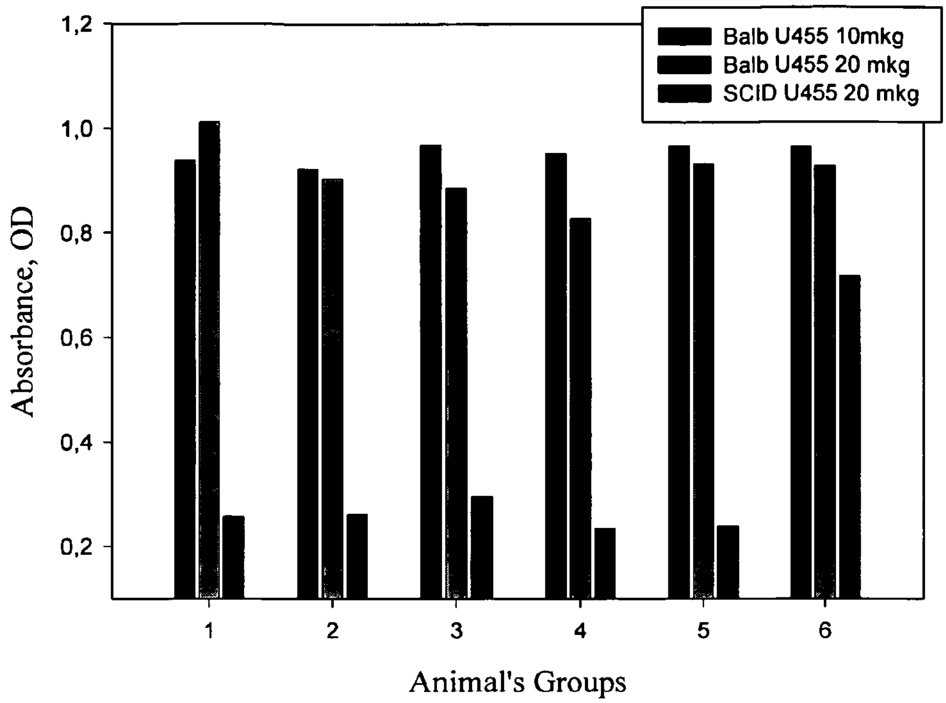


**Figure 2: SCID-PBMC and BalbC Immune Response for Immunization with Coctails of Recombinant p120-1 and p120-2 Proteins**

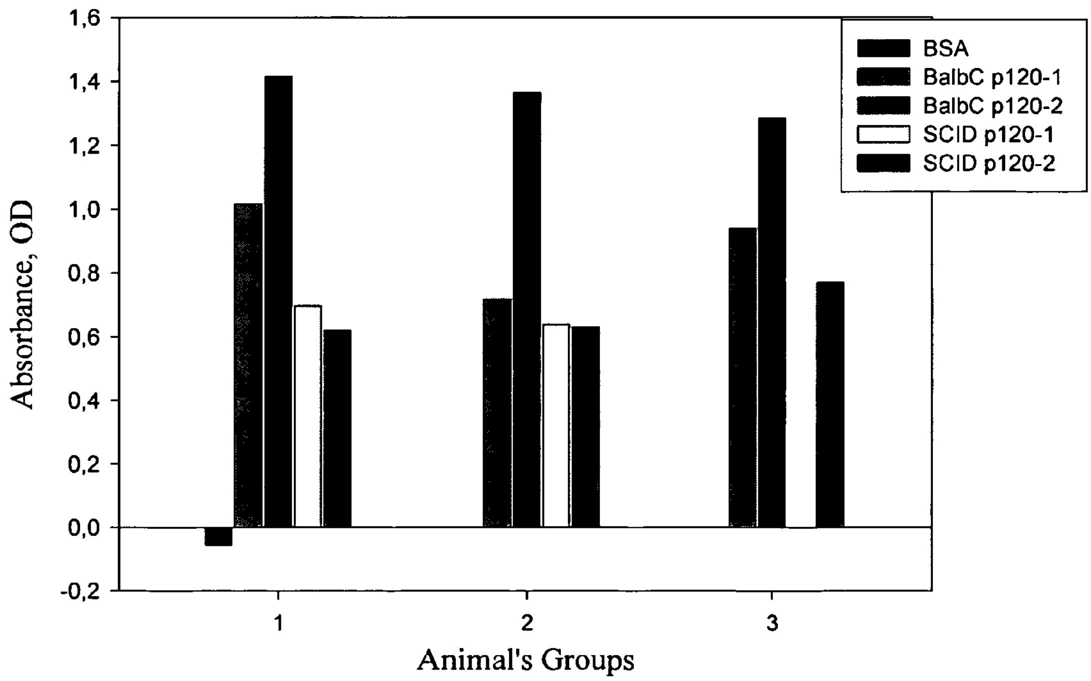

**Figure 3: PokA-79 Infection Rates on U937 and MT-4 Background, 3 Challenges 7 Days b/w Period**

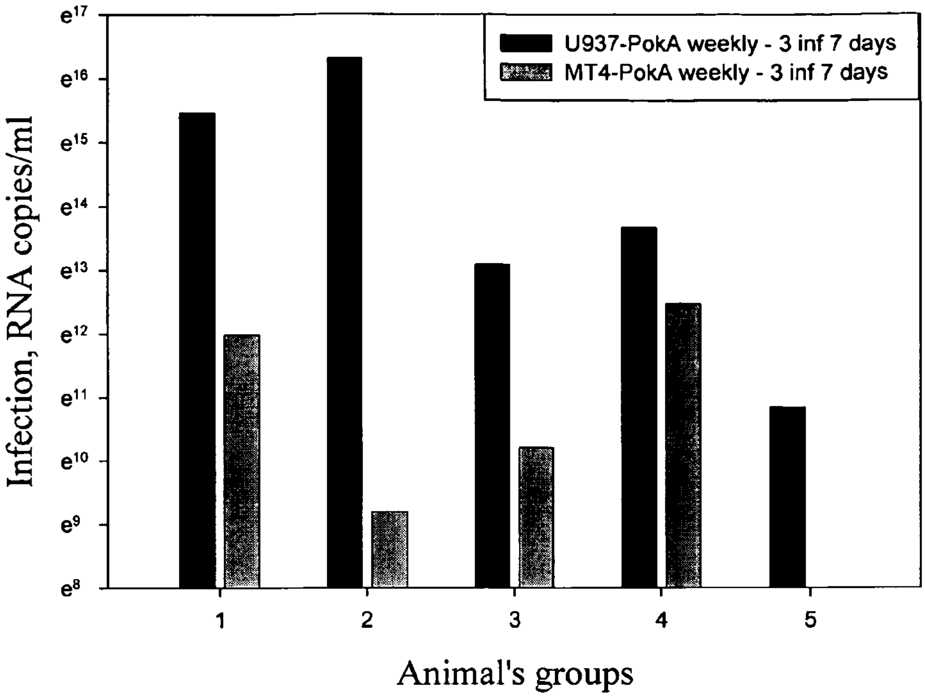


**Figure 4: PokA-79 Infection Rates on U937 and MT-4 Background, 2 Challenges 3 Days b/w Period**

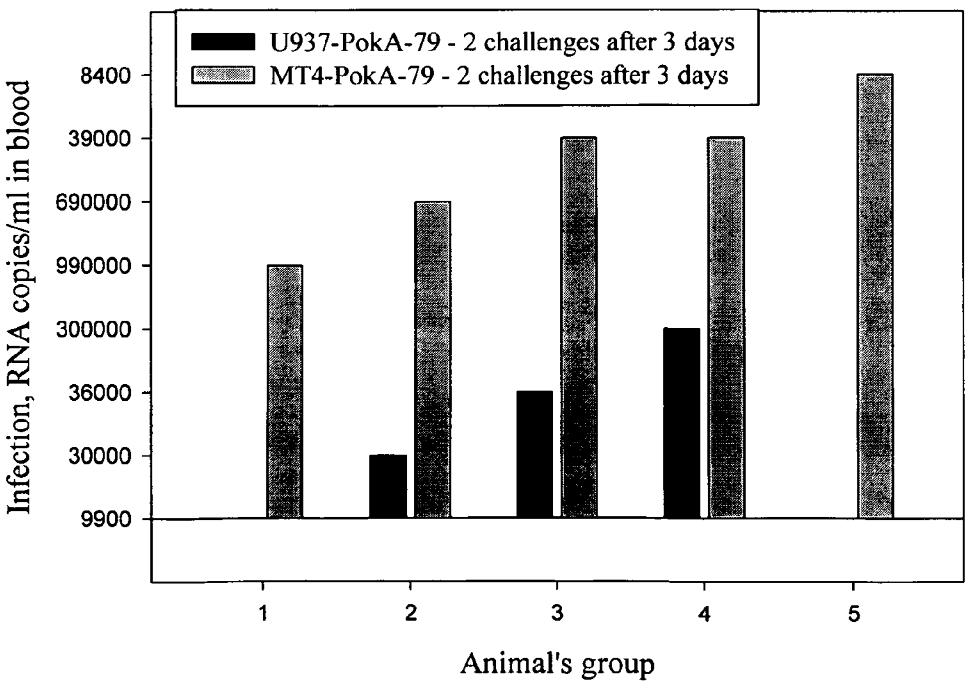

**Figure 5: PokA-79 - MT4 and U937-background's Infection Rates for Different Periods between Challenging in SCID Mice**

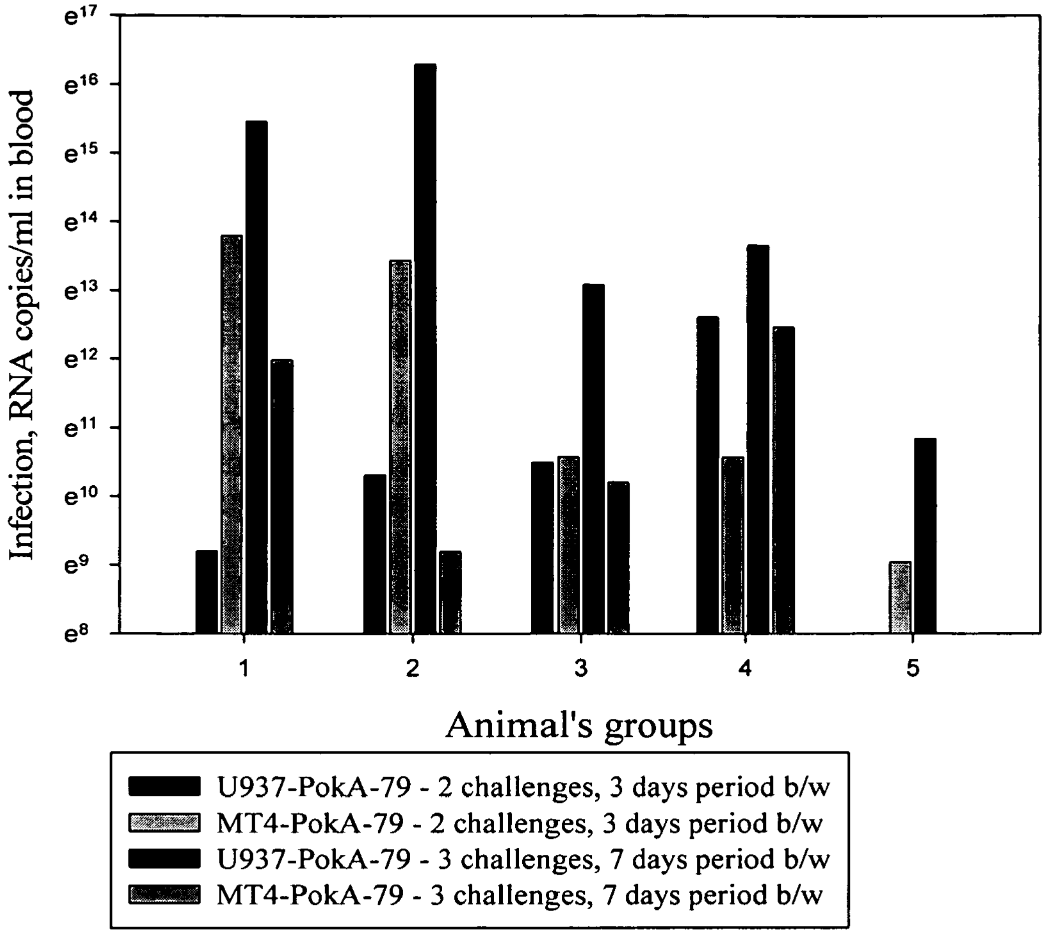

**Figure 6: PokA-79 in vitro Replication Dynamics, Lymphocyte MT-4 and Macrophage U937 Background**

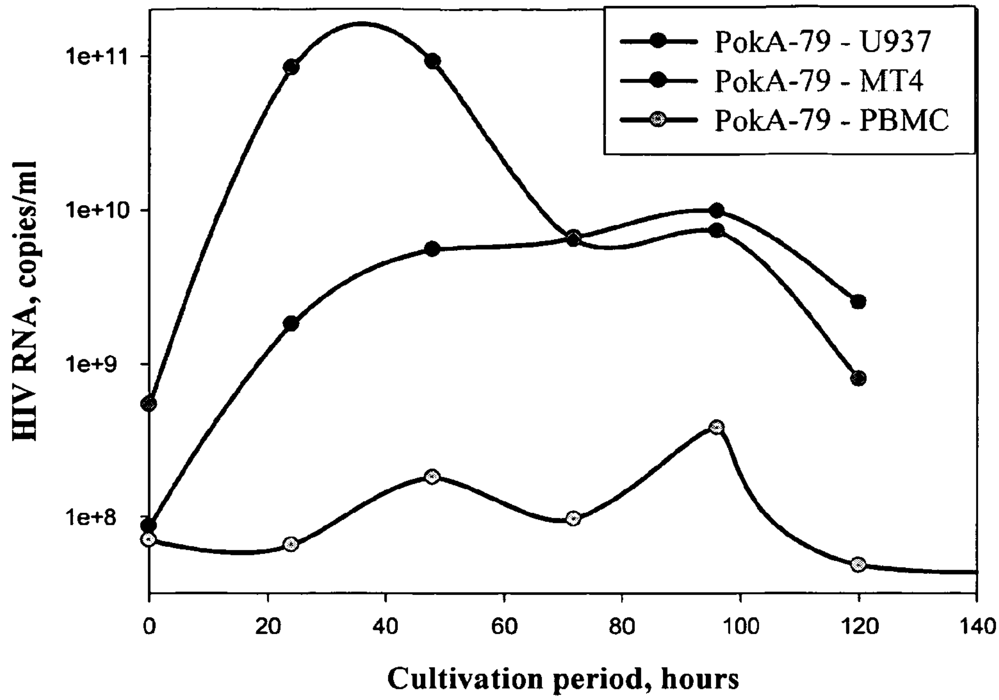


**Figure 7: PokA-79 in vitro Replication Dynamics, p24 Expression, Lymphocyte MT-4 and Macrophage U937 Background**

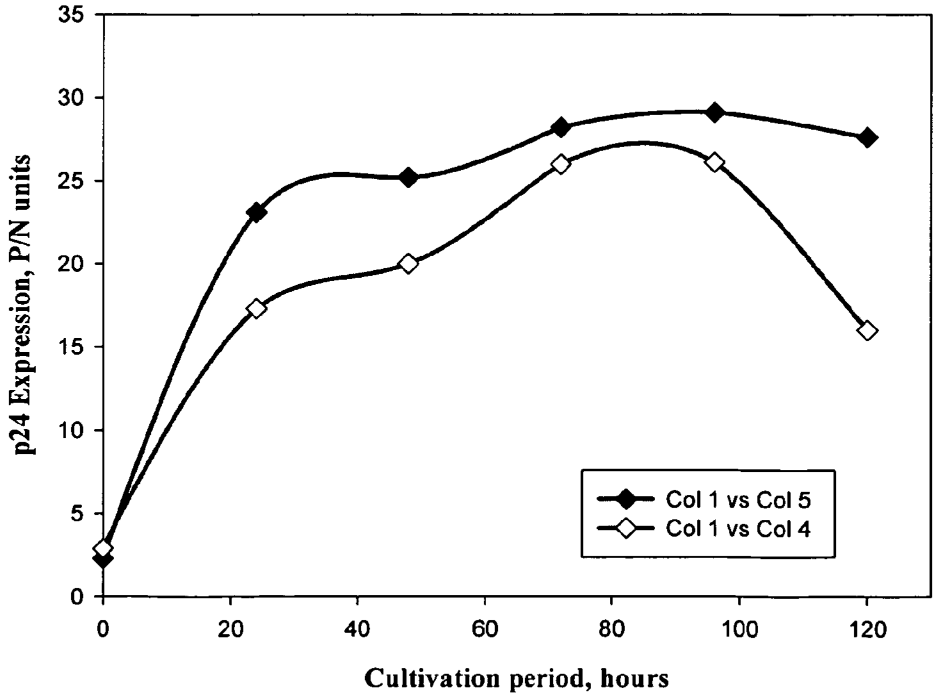

**Figure 8: Laboratory Strains U455 and IIIB/H9 Infection Rates on MT-4 and U937 Cell Cultures background, 4 Challenges with 7 Days b/w Period**

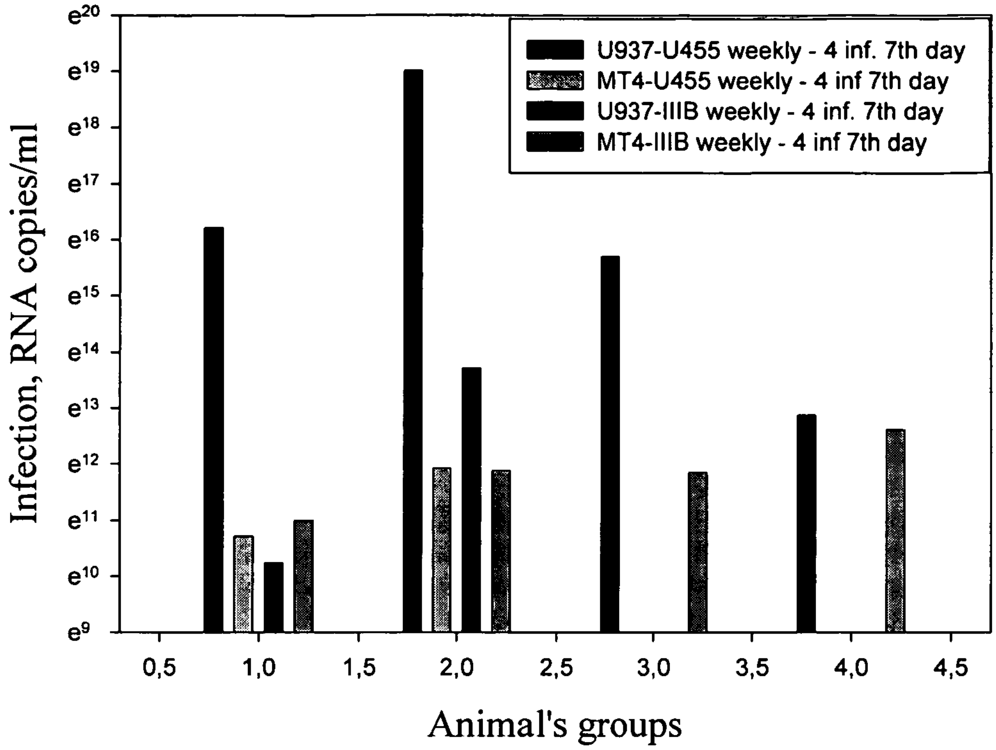

**Figure 9: U455 and H9/IIIB in vitro Replication Dynamicson Lymphocytes MT-4 and Macrophage U937 Background**

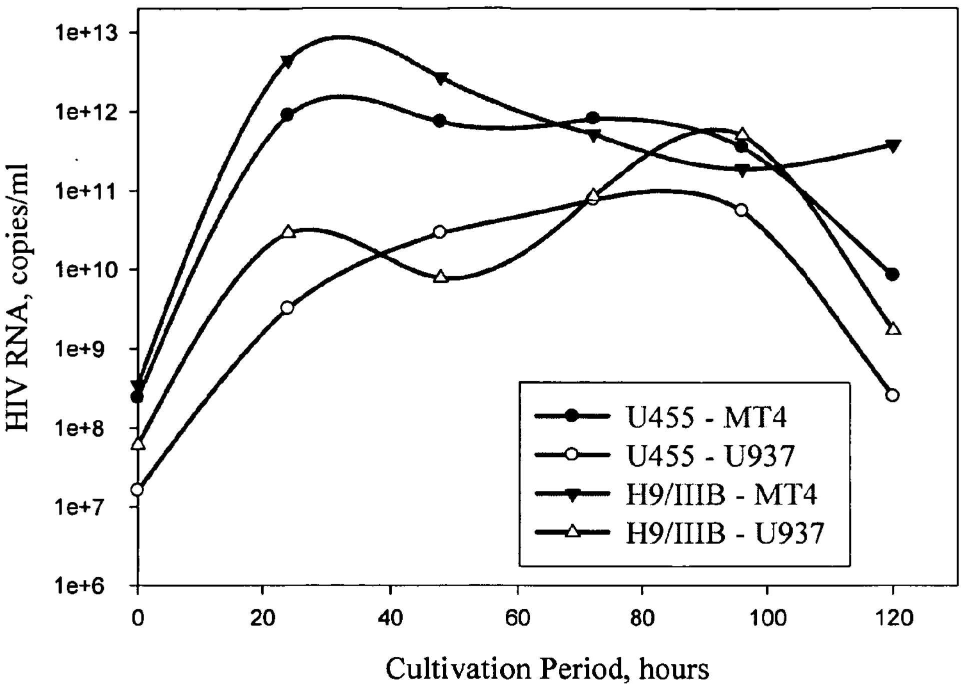


**Figure 10: U455 and H9/IIIB in vitro Replication Dynamics, p24 Expression on Lymphocyte MT-4 and Macrophage U937 Cultures**

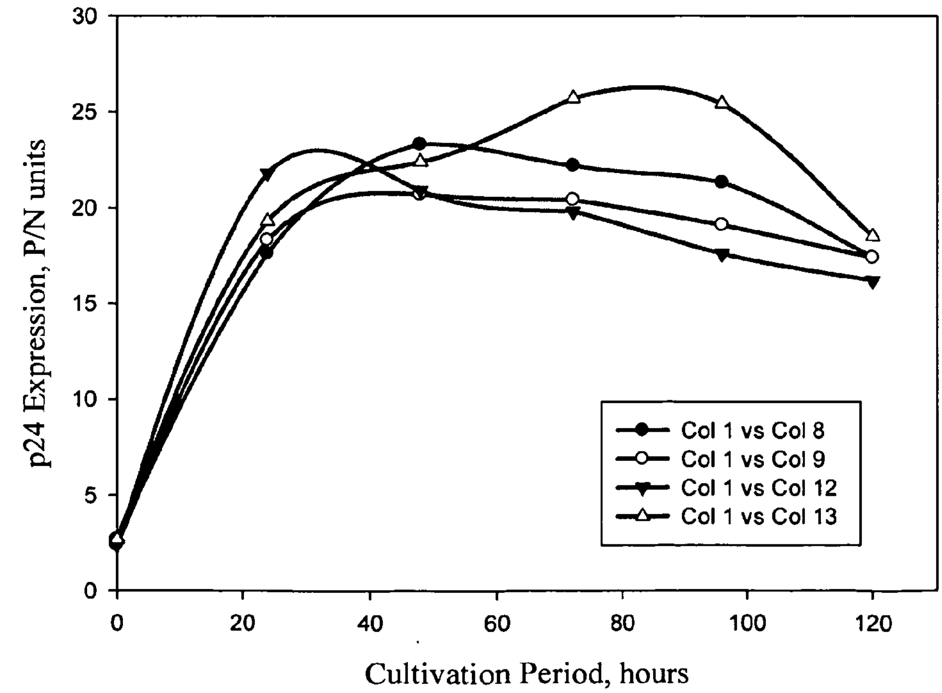

**Figure 11: PokA-79 Infection Rates on Cell Culture U937 and MT4 Background Depending on the Number of Challenging**

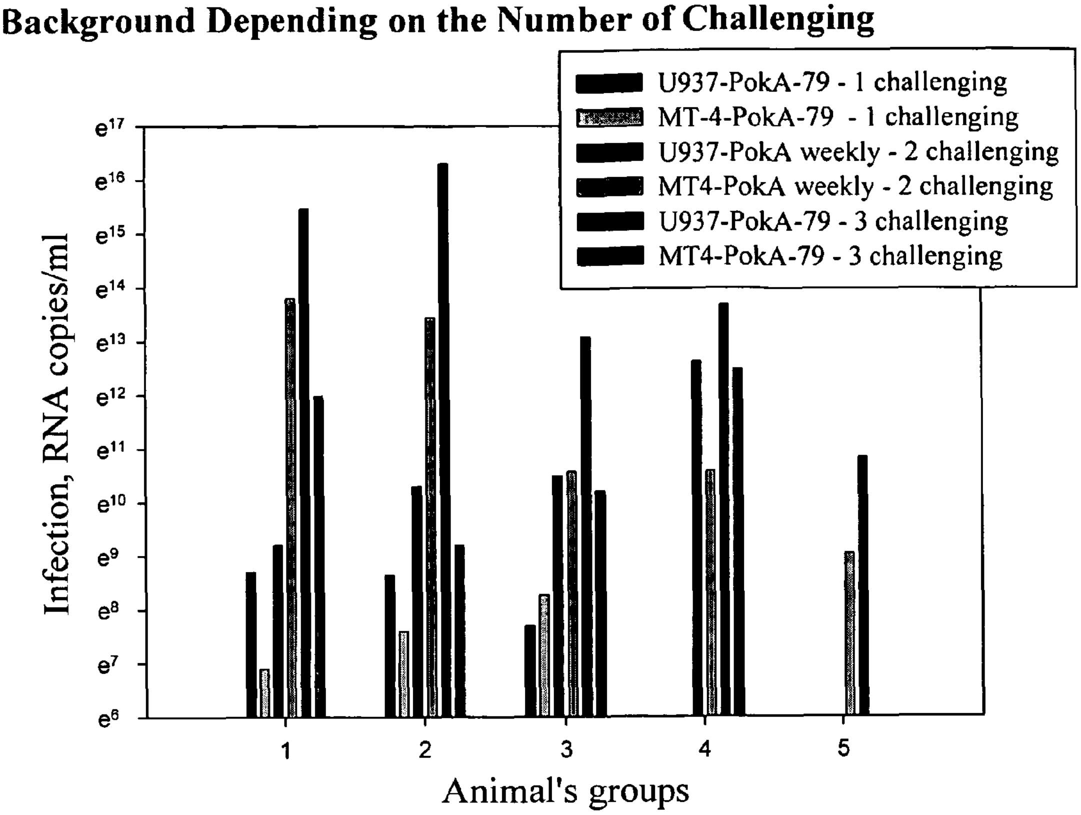


**Figure 12: SCID-PBMC and SCID-cell Culture Background Infection Rates for Challenging with PokA-79 Laboratory Strain**

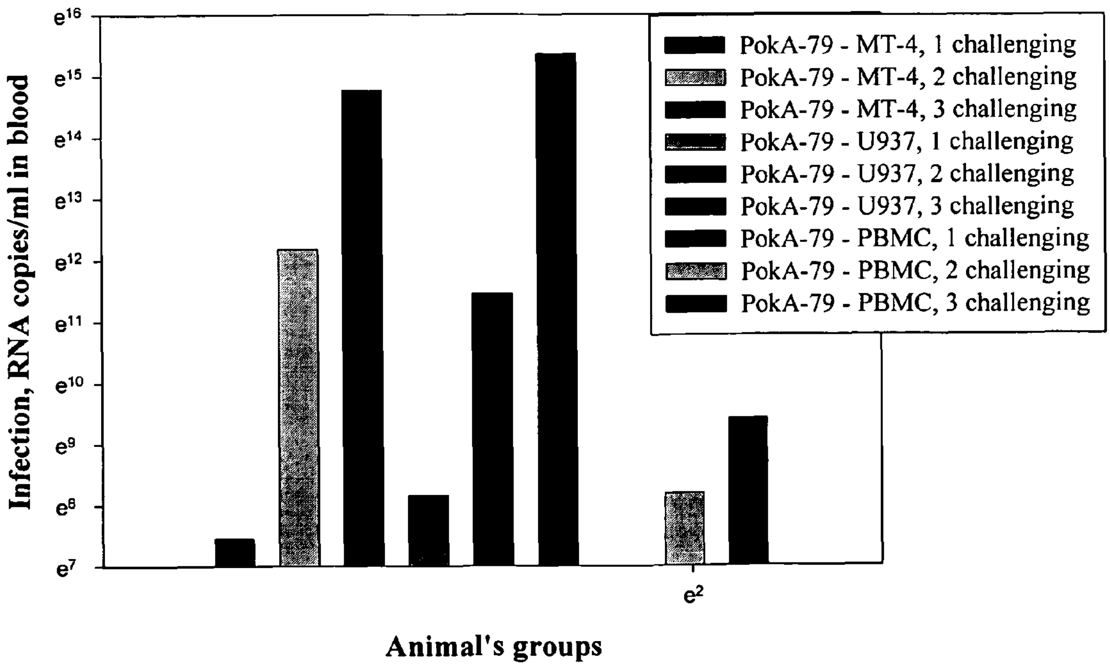

**Figure 13: Recombinant p120-1 and p120-2 Immunization Effect for SCID-PBMC Challenging with PokA-79-MT-4 Laboratory Strain**

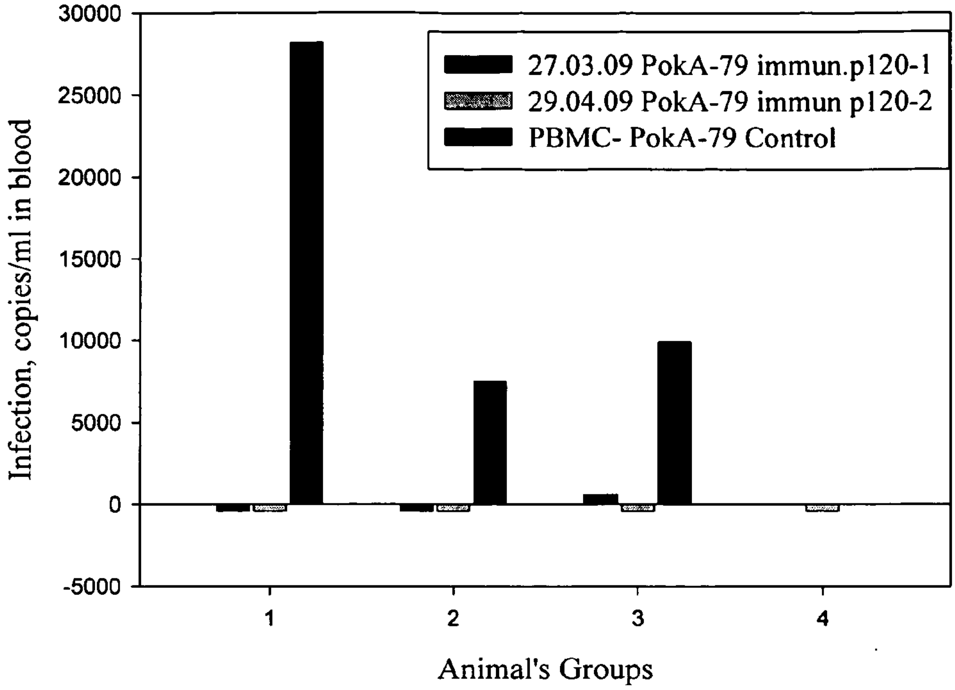


**Figure 14: Immune Responses for p120-1 and p120-2 in Challenged with PokA-79 - MT-4 Laboratory Strain SCID-PBMC Mice**

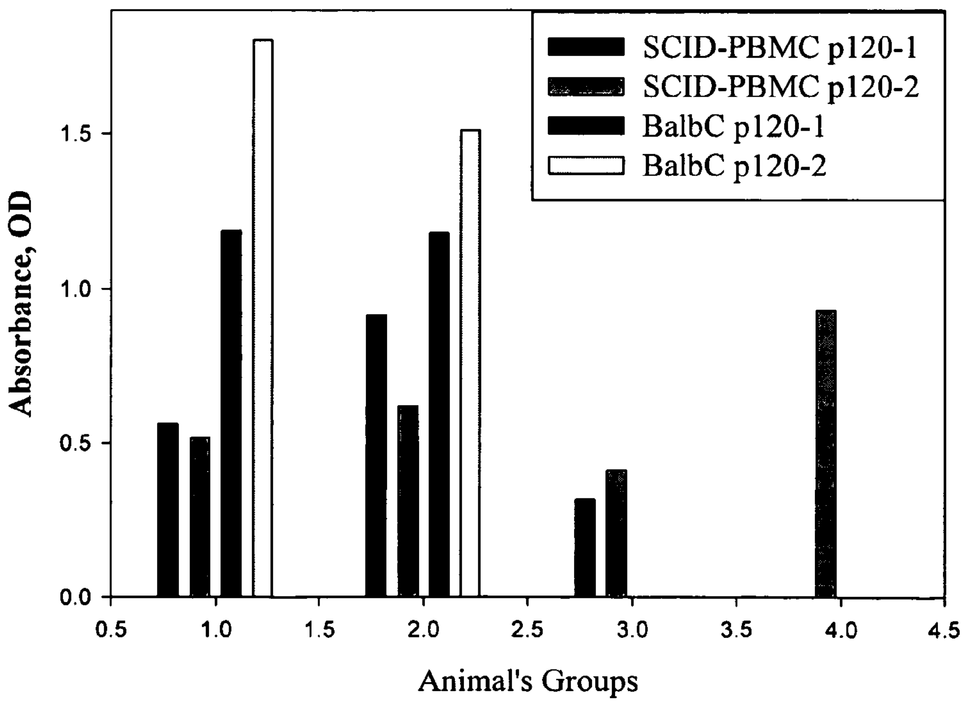

Europäisches Patentamt
European Patent Office
Office européen des brevets

# EUROPEAN SEARCH REPORT

Application Number
EP 10 00 8952

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2004/109876 A1 (YAMAMOTO NAOKI [JP] ET AL) 10 June 2004 (2004-06-10)<br>* paragraph [0028] - paragraph [0068] *<br>----- | 1-10, 12-17, 19,20 | INV. A01K67/027 A61K49/00 |
| X | WO 98/44788 A2 (CHANG LUNG JI [US]) 15 October 1998 (1998-10-15)<br>* page 16 - page 18; claims 16-31; example 21 *<br>----- | 1-3,7, 9-13, 16-18 | |
| X | WO 2008/010099 A2 (PASTEUR INSTITUT [FR]; CENTRE NAT RECH SCIENT [FR]; UNIV PARIS DESCART) 24 January 2008 (2008-01-24)<br>* claims 29,30,32; example 2 *<br>----- | 1,2,7,9, 10 | |
| A | WO 2008/094178 A2 (VAXDESIGN CORP [US]; UNIV VIRGINIA COMMONWEALTH [US]; TEW JOHN G [US];) 7 August 2008 (2008-08-07)<br>* page 7 - page 11 *<br>----- | 1-20 | |
| A | WO 2009/105244 A1 (UNIV NORTH CAROLINA [US]; SU LISHAN [US]; ZHANG LIGUO [US]; WASHBURN M) 27 August 2009 (2009-08-27)<br>* claims 1-7 *<br>----- | 1-20 | TECHNICAL FIELDS SEARCHED (IPC)<br>A01K<br>A61K |
| A | VAN DUYNE RACHEL ET AL: "The utilization of humanized mouse models for the study of human retroviral infections", RETROVIROLOGY, BIOMED CENTRAL LTD., LONDON, GB LNKD- DOI:10.1186/1742-4690-6-76, vol. 6, no. 1, 12 August 2009 (2009-08-12), page 76, XP021059355, ISSN: 1742-4690<br>* page 6 - page 11 *<br>-----<br>-/-- | 1-20 | |

The present search report has been drawn up for all claims

| Place of search | Date of completion of the search | Examiner |
|---|---|---|
| Munich | 12 November 2010 | Deleu, Laurent |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

Europäisches Patentamt
European Patent Office
Office européen des brevets

# EUROPEAN SEARCH REPORT

Application Number
EP 10 00 8952

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A | SHULTZ L D ET AL: "Humanized mice in translational biomedical research", NATURE REVIEWS. IMMUNOLOGY, NATURE PUBLISHING GROUP, GB LNKD- DOI:10.1038/NRI2017, vol. 7, no. 2, 1 February 2007 (2007-02-01), pages 118-130, XP002493022, ISSN: 1474-1733 * page 124 * ----- | 1-20 | |
| | | | TECHNICAL FIELDS SEARCHED (IPC) |

The present search report has been drawn up for all claims

2

| Place of search | Date of completion of the search | Examiner |
|---|---|---|
| Munich | 12 November 2010 | Deleu, Laurent |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 10 00 8952

This annex lists the patent family members relating to the patent documents cited in the above-mentioned European search report.
The members are as contained in the European Patent Office EDP file on
The European Patent Office is in no way liable for these particulars which are merely given for the purpose of information.

12-11-2010

| Patent document cited in search report | Publication date | Patent family member(s) | Publication date |
|---|---|---|---|
| US 2004109876 A1 | 10-06-2004 | NONE | |
| WO 9844788 A2 | 15-10-1998 | AU 752753 B2 | 26-09-2002 |
| | | AU 6890998 A | 30-10-1998 |
| | | CA 2284689 A1 | 15-10-1998 |
| | | EP 0973381 A2 | 26-01-2000 |
| | | JP 4312265 B2 | 12-08-2009 |
| | | JP 2002501375 T | 15-01-2002 |
| WO 2008010099 A2 | 24-01-2008 | CA 2657498 A1 | 24-01-2008 |
| | | EP 1878798 A1 | 16-01-2008 |
| | | EP 2041290 A2 | 01-04-2009 |
| | | JP 2009542252 T | 03-12-2009 |
| | | US 2010138938 A1 | 03-06-2010 |
| WO 2008094178 A2 | 07-08-2008 | AU 2007345747 A1 | 07-08-2008 |
| | | CA 2655344 A1 | 07-08-2008 |
| | | EP 2043695 A2 | 08-04-2009 |
| | | US 2008008653 A1 | 10-01-2008 |
| WO 2009105244 A1 | 27-08-2009 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0937389 A2
- US 2002001798 A1
- WO 9912553 A1
- WO 2009046984 A1
- US 20070978536P A
- WO 2001082964 A
- US 2007011753 A1
- WO 2005040353 A
- WO 2006031264 A
- WO 9405779 A1
- WO 03106479 A2


### Non-patent literature cited in the description


- **BONYHADI ML ; KANESHIMA H.** The HuSCID Mouse: an in vivo Model for HIV Infection in Humans. *Molecular medicine today,* June 1997, 246-253
- **BRAINARD D.M ; SEUNG E. ; FRAHM N. ; CARIAPPA A. ; BAILEY C.C. et al.** Induction of Robust Cellular and Humoral Virus-Specific Adaptive Immune Responses in Human Immunodeficiency Virus-Infected Humanized BLT Mice. *J. Virology,* July 2009, vol. 83 (14), 7305-7321
- **CAPODICI J. et al.** Large-Scale Beta-Propiolactone Inactivation of HIV for Vaccines. *Bioprocess Int.,* February 2006, 36-41
- Animal Model Systems of HIV-Diseases. **FRIEDMAN H. ; SPECTER S. ; BENDINELLI M.** In vivo Models of HIV Disease and Control. Springer, 2006
- **LAPENTA C ; FAIS S ; RIZZA P ; SPADA M ; LOGOZZI MA et al.** U937-SCID mouse xenografts: a new model for acute in vivo HIV-1 infection suitable to test antiviral strategies. *Antiviral Research,* 1997, vol. 36, 81-90
- **MOSIER DE ; GULIZIA RJ ; BAIRD SM ; WILSON DB.** Transfer of a functional human immune system to mice with severe combined immunodeficiency. *Nature,* 15 September 1988, vol. 335 (6187), 256-9
- **POIGNARD P.** Heterogeneity of envelope molecules expressed on primary human immunodeficiency virus type 1 particles as probed by the binding of neutralizing and non-neutralizing antibodies. *J. Viro1.,* January 2003, vol. 77 (1), 353-65
- **STEINBROOK R.** One Step Forward, Two Steps Back - Will There Ever Be an AIDS Vaccine?. *New England Journal of Medicine,* 27 December 2007, vol. 357 (26), 2653-2655
- **STODDART C.A ; BALES C.A ; BARE J.C ; CHKHENKELI G. ; GALKINA S.A. et al.** Validation of the HuSCID Thy/Liv Mouse Model with Four Classes of Licensed Antiretrovirals. *PLoS,* August 2007, 655
- **SU L. ; KANESHIMA H. ; BONYHADI M. ; SALIMI S. ; KRAFT D. ; RABIN L. ; MCCUNE J. M.** HIV-1 -Induced Thymocyte Depletion Is Associated with Indirect Cytopathicity and Infection of Progenitor dells In Vivo. *Immunity,* January 1995, vol. 2, 25-36
- **WILLIAM C. ; GRIFFIN WC ; MIDDAUGH LD ; COOK JE ; TYOR WR.** The severe combined immunodeficient (SCID) mouse model of human immunodeficiency virus encephalitis: Deficits in cognitive function. *Journal of Neurovirology,* April 2004, vol. 10 (2), 109-115
- **VAN DUYNE R ; PEDATI C ; GUENDEL I. ; CARPIO L ; KEHN-HALL K ; SAIFUDDIN M ; KASHANCHI F.** The utilization of humanized mouse models for the study of human retroviral infections. *Retrovirology,* 2009, vol. 6, 76
- **FIKHTENGHOLTZ G.M.** Differential and Integral Math Analysis. 1969, vol. 2
- **WOLKENSTEIN M.V.** Molecular Biophysics. Nauka, 1975
- **JONULEIT H ; KUHN U ; MULLER G ; STEINBRINK K ; PARAGNIK L ; SCHMITT E ; KNOP J ; ENK AH.** Pro-inflammatory cytokines and prostaglandins induce maturation of potent immunostimulatory dendritic cells under fetal calf serum-free conditions. *Eur J Immunol.,* December 1997, vol. 27 (12), 3135-42